# EUROPEAN PATENT APPLICATION

(11) **EP 4 074 380 A1**
(43) Date of publication of application: **19.10.2022**
(21) Application number: 20905471.7
(22) Date of filing: 25.12.2020
(51) Int. Cl.: A61Q 17/00, A61Q 19/00, C07K 5/06, C07K 5/08, C07K 5/10, C09K 3/00, A61Q 5/00, A61K 8/64

(54) **ANTI-POLLUTION MATERIAL**

(30) Priority: 26.12.2019 JP 2019236433
(71) Applicant: Nissan Chemical Corporation, Tokyo 103-6119 (JP)
(72) Inventor: SAKATA, Mizuki, Funabashi-shi, Chiba 274-0052 (JP)
(74) Representative: Hoffmann Eitle
(86) International application number: PCT/JP2020/048971
(87) International publication number: WO 2021/132653

(57) **Abstract**

There is provided a new material for preventing adhesion of pollutants by forming a film on skin or on the surface of hair to prevent the skin and surface of hair from being polluted due to the pollutants. Provided is an anti-pollution material characterized by containing a lipid-peptide compound in which a peptide moiety formed from repetition of at least two amino acids which are the same or different binds to a lipid moiety comprising an aliphatic group having 10-24 carbon atoms.

## Description

### TECHNICAL FIELD

The present invention relates to an anti-pollution material, and more particularly to a material that prevents adhesion of dust, pollen, particulate matter, etc. by forming a film on the surface of skin or hair.

### BACKGROUND ART

In recent years, there has been growing interest in allergens (e.g., pollen) and harmful substances such as fine particulate matter (e.g., PM 2.5), and various products including masks have been developed to prevent these substances from being taken into the human body.

One of such proposed products is a product for preventing harmful substances from adhering to the body, clothes, etc. through spraying or the like. For example, there have been proposed a pollen adsorption inhibitor containing a polymer containing, as a constituent unit, a monomer unit having a specific amphoteric ion group or anionic group (Patent Document 1), as well as a harmful substance adhesion inhibitor containing hydroxyalkyl chitosan (Patent Document 2).

In the field of cosmetics and hairdressing products, a film formed on the skin or the surface of hair provides an effective barrier on the skin or the hair surface, and thus the film plays various important roles in personal care products, such as inhibiting evaporation of moisture in the skin or hair, and enhancing percutaneous absorption or retention of active ingredients to be penetrated into the hair.

For example, skin care formulations that exhibit a high moisturizing effect include coating cosmetic products having a self-assembled structure, such as those utilizing layered α-gel (Patent Document 3).

Also, hair care formulations utilizing polypeptides have been proposed for reducing hair damage caused by brushing or heat treatment with a dryer or the like (Patent Documents 4 and 5).

### Prior Art Documents

### Patent Documents

Patent Document 1: JP 4562585 B
Patent Document 2: JP 6198799 B
Patent Document 3: JP 2016-6030 A
Patent Document 4: JP 1998-77210 A
Patent Document 5: JP 2002-308756 A

### SUMMARY OF THE INVENTION

### Problems to be Solved by the Invention

Hitherto, there has been no report on a material that prevents adhesion of dust, pollen, particulate matter, etc. to the surface of hair or skin by means of a gel-like substance using a polypeptide.

An object of the present invention is to provide a new material that forms a film on the skin or the surface of hair, to thereby prevent adhesion of a pollutant to the skin or the hair surface, and to prevent pollution of the skin or the hair surface with the pollutant.

### Means for Solving the Problems

The present inventor has found that when a film is formed on the skin or the surface of hair from a material containing at least one lipidic peptide compound, the skin or the hair surface is prevented from being polluted with a pollutant. The present invention has been accomplished on the basis of this finding.

Accordingly, a first aspect of the present invention is an anti-pollution material characterized by comprising a lipidic peptide compound wherein a lipidic moiety having a C₁₀₋₂₄ aliphatic group is bonded to a peptide moiety formed of at least two identical or different amino acid repeats.

A second aspect of the present invention is the anti-pollution material according to the first aspect, wherein the anti-pollution material can form an anti-pollution film on the surface of skin or hair.

A third aspect of the present invention is the anti-pollution material according to the second aspect, wherein the anti-pollution material prevents adhesion of dust, pollen, particulate matter, mites (including carcasses thereof), gaseous matter, or odorous matter to the surface of skin or hair.

A fourth aspect of the present invention is the anti-pollution material according to any one of the first to third aspects, wherein the lipidic peptide compound contains at least one of compounds of the following Formulae (1) to (3) or pharmaceutically usable salts of the compounds: 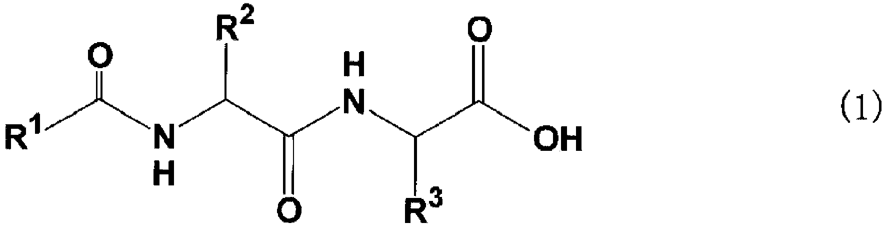 (wherein R¹ is a C₉₋₂₃ aliphatic group; R² is a hydrogen atom, or a C₁₋₄ alkyl group having, if necessary, a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring group having, if necessary, one to three nitrogen atoms, or a fused heterocyclic ring group composed of the 5-membered ring and the 6-membered ring), 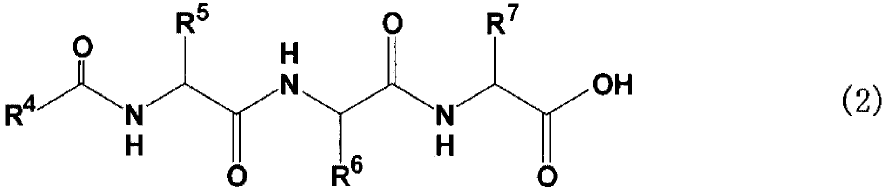 (wherein R⁴ is a C₉₋₂₃ aliphatic group; and R⁵ to R⁷ are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring), and 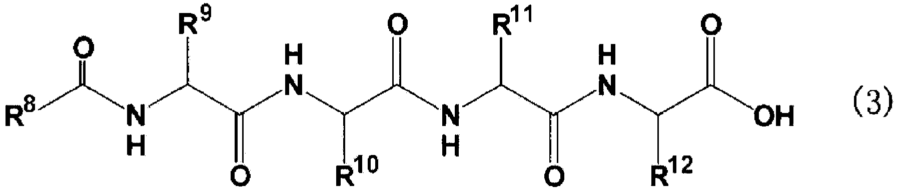 (wherein R⁸ is a C₉₋₂₃ aliphatic group; and R⁹ to R¹² are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring).

A fifth aspect of the present invention is a method for preventing pollution of the surface of skin or hair, the method comprising a film formation step of forming a film, on the surface of skin or hair, from an anti-pollution material containing a lipidic peptide compound wherein a lipidic moiety having a C₁₀₋₂₄ aliphatic group is bonded to a peptide moiety formed of at least two identical or different amino acid repeats.

A sixth aspect of the present invention is the method according to the fifth aspect, wherein the lipidic peptide compound contains at least one of compounds of Formulae (1) to (3) or pharmaceutically usable salts of the compounds.

A seventh aspect of the present invention is a method for preventing adhesion of dust, pollen, mites (including carcasses thereof), particulate matter, gaseous matter, or odorous matter to the surface of skin or hair, the method comprising a film formation step of forming a film, on the surface of skin or hair, from an anti-pollution material containing a lipidic peptide compound wherein a lipidic moiety having a C₁₀₋₂₄ aliphatic group is bonded to a peptide moiety formed of at least two identical or different amino acid repeats.

An eighth aspect of the present invention is the method according to the seventh aspect, wherein the lipidic peptide compound contains at least one of compounds of Formulae (1) to (3) or pharmaceutically usable salts of the compounds.

A ninth aspect of the present invention is the anti-pollution material according to any one of the first to fourth aspects, wherein a film formed from the material has a surface roughness, and the average surface roughness is 3 nm to 500 nm.

### Effects of the Invention

According to the present invention, formation of a film on the surface of skin or hair from an anti-pollution material containing a specific lipidic peptide compound can prevent adhesion of dust, pollen, particulate matter, etc. to the skin or hair surface, to thereby prevent pollution of the skin or the hair with such a substance.

According to the present invention, the aforementioned anti-pollution material can prevent adhesion of dust, pollen, particulate matter, etc., to thereby inhibit, for example, skin irritation that may be caused by such a substance, and to avoid skin troubles such as inflammation, oxidation, and skin aging.

Furthermore, according to the present invention, adhesion of fine particulate matter such as pollen or PM 2.5 can be prevented, to thereby prevent, for example, introduction of pollutants into houses, etc. or inhalation of the pollutants, which may be caused by moving around with the pollutants attached to the skin or hair. This can be expected to reduce occurrence of allergic symptoms or similar symptoms due to such pollutants.

According to the present invention, adhesion of unpleasant odors (e.g., cigarette smoke or odorants) to the surface of skin or hair can be prevented.

The lipidic peptide compound used in the anti-pollution material of the present invention is a highly safe artificial low-molecular-weight compound that is composed of a lipid and a peptide only. Thus, the material of the present invention has high biological safety, and is very useful for the aforementioned applications, from the viewpoint of high safety required in pharmaceutical products and cosmetic applications. Since the present invention involves the use of a film formed from a low-molecular-weight lipidic peptide compound, a good sensation in use can be achieved during formation of the film on the skin or hair through coating or the like, as compared with a conventionally proposed product for preventing adsorption or adhesion of pollen, etc.

### BRIEF DESCRIPTION OF THE DRAWINGS

[FIG. 1] FIG. 1 shows the results of a test for adhesion of carbon ECF-600JD .
[FIG. 2] FIG. 2 shows the results of observation in a test for adhesion of cedar pollen to an artificial skin model BIOSKIN.
[FIG. 3] FIG. 3 shows the results of observation with a scanning electron microscope (SEM) in a test for adhesion of cedar pollen to an artificial skin model BIOSKIN.
[FIG. 4] FIG. 4 shows the results of quantification of Cryj 1 (cedar pollen allergen) in a test for adhesion of cedar pollen to an artificial skin model BIOSKIN.
[FIG. 5] FIG. 5 shows the results of observation in a test for adhesion of cedar pollen to an artificial leather SUPPLALE.
[FIG. 6] FIG. 6 shows the results of quantification of Cryj 1 (cedar pollen allergen) in a test for adhesion of cedar pollen to an artificial leather SUPPLALE.
[FIG. 7] FIG. 7 shows the results of observation in a test for adhesion of PM 2.5 particles to an artificial leather SUPPLALE.
[FIG. 8] FIG. 8 shows the results of determination of the amount of PM 2.5 particles adhered to an artificial leather SUPPLALE, wherein the amount of adhesion is converted into area percentage through image processing with a microscope.
[FIG. 9] FIG. 9 shows the results of determination of the amount of PM 2.5 particles adhered to an artificial leather SUPPLALE with an ICP-emission spectrophotometer.
[FIG. 10] FIG. 10 shows the results of determination of the amount of PM 2.5 particles adhered to an artificial leather SUPPLALE with an energy dispersive X-ray fluorescence analyzer.
[FIG. 11] FIG. 11 shows the results of observation with a microscope in a test for adhesion of PM 2.5 particles to an artificial leather SUPPLALE.
[FIG. 12] FIG. 12 shows the results of determination of the amount of PM 2.5 particles adhered to an artificial leather SUPPLALE, wherein the amount of adhesion is converted into area percentage through image processing with a microscope.
[FIG. 13] FIG. 13 shows an apparatus used for spraying of PM 2.5 particles to an artificial leather SUPPLALE.
[FIG. 14] FIG. 14 shows the results of determination (with an energy dispersive X-ray fluorescence analyzer) of the amount of Si adhered to an artificial leather SUPPLALE through spraying of PM 2.5 particles thereto.
[FIG. 15] FIG. 15 shows the results of determination of the amount of PM 2.5 particles adhered to an artificial leather SUPPLALE with an ICP-emission spectrophotometer.
[FIG. 16] FIG. 16 shows the results of observation with a scanning electron microscope (SEM) of a film formed through coating of an anti-pollution material containing a lipidic peptide compound.
[FIG. 17] FIG. 17 shows the results of quantification of Derf1 (mite allergen) in a test for adhesion of mites to an artificial leather SUPPLALE.
[FIG. 18] FIG. 18 shows the results of observation in a test for adhesion of mites to an artificial leather SUPPLALE.
[FIG. 19] FIG. 19 shows the results of measurement of the roughness of a film formed on the surface of a silicon wafer with an atomic force microscope (AFM).
[FIG. 20] FIG. 20 shows the results of quantification of Cryj 1 (cedar pollen allergen) in a test for adhesion of cedar pollen to an artificial leather SUPPLALE.

### MODES FOR CARRYING OUT THE INVENTION

The present invention is directed to an anti-pollution material containing a specific lipidic peptide compound.

The anti-pollution material of the present invention can form a film on the surface of skin or hair, to thereby prevent adhesion of a pollutant (e.g., dust) to the skin and hair surface and to exhibit the effect of preventing pollution caused by such a pollutant (i.e., anti-pollution effect).

Examples of the matter targeted for prevention of adhesion or pollution to the surface of skin or hair include dust, pollen, air pollutants such as exhaust gas and factory smoke, particulate matter that may be contained in cigarette smoke, etc. (e.g., PM 10, suspended particulate matter (SPM), and PM 2.5 (fine particulate matter)), gaseous matter (e.g., SOx and CO), odorous matter, allergens such as house dust and fungi, mites (including carcasses thereof), and viruses such as influenza virus.

Ingredients of the anti-pollution material will next be described.

### [Anti-Pollution Material]

### [Lipidic Peptide Compound]

The lipidic peptide compound usable in the anti-pollution material of the present invention, wherein a lipidic moiety having a C₁₀₋₂₄ aliphatic group (the entire lipidic moiety has a carbon atom number of 10 to 24) is bonded to a peptide moiety formed of at least two identical or different amino acid repeats, is any of compounds of the following Formulae (1) to (3) (lipidic peptides) or pharmaceutically usable salts of the compounds (low-molecular-weight compounds each having a lipidic moiety as a hydrophobic moiety and a peptide moiety as a hydrophilic moiety).

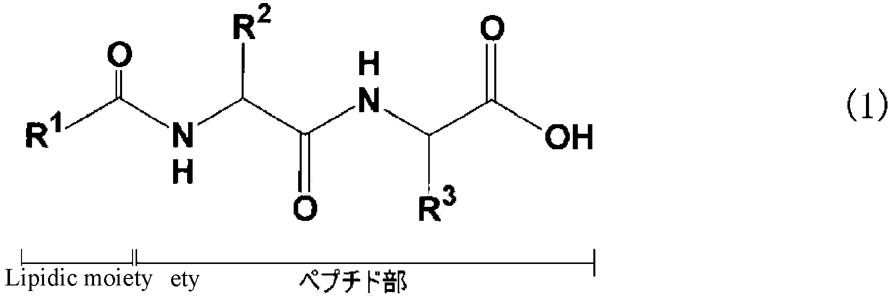

In Formula (1), R¹ is a C₉₋₂₃ aliphatic group. Preferably, R¹ is a linear aliphatic group having a carbon atom number of 11 to 23 and having, if necessary, zero to two unsaturated bonds.

Specific examples of the lipidic moiety (acyl group) composed of R¹ and the adjacent carbonyl group include lauroyl group, dodecylcarbonyl group, myristoyl group, tetradecylcarbonyl group, palmitoyl group, margaroyl group, oleoyl group, elaidoyl group, linoleoyl group, stearoyl group, vaccenoyl group, octadecylcarbonyl group, arachidoyl group, eicosylcarbonyl group, behenoyl group, elkanoyl group, docosylcarbonyl group, lignoceroyl group, and nervonoyl group. Particularly preferred examples include lauroyl group, myristoyl group, palmitoyl group, margaroyl group, stearoyl group, oleoyl group, elaidoyl group, and behenoyl group.

In Formula (1), R² included in the peptide moiety is a hydrogen atom or a C₁₋₄ alkyl group having, if necessary, a C₁ or C₂ branched chain.

The C₁₋₄ alkyl group having, if necessary, a C₁ or C₂ branched chain refers to an alkyl group having a C₁₋₄ main chain and having, if necessary, a C₁ or C₂ branched chain. Specific examples of the alkyl group include methyl group, ethyl group, n-propyl group, i-propyl group, n-butyl group, i-butyl group, sec-butyl group, and tert-butyl group.

R² is preferably a hydrogen atom or a C₁₋₃ alkyl group having, if necessary, a C₁ branched chain, more preferably a hydrogen atom.

The C₁₋₃ alkyl group having, if necessary, a C₁ branched chain refers to an alkyl group having a C₁₋₃ main chain and having, if necessary, a C₁ branched chain. Specific examples of the alkyl group include methyl group, ethyl group, n-propyl group, i-propyl group, i-butyl group, and sec-butyl group. Preferred is methyl group, i-propyl group, i-butyl group, or sec-butyl group.

In Formula (1), R³ is a -(CH₂)ₙ-X group. In the -(CH₂)ₙ-X group, n is a number of 1 to 4, and X is an amino group, a guanidino group, a -CONH₂ group, or a 5-membered ring or 6-membered ring group having, if necessary, one to three nitrogen atoms, or a fused heterocyclic ring group composed of the 5-membered ring and the 6-membered ring.

In the -(CH₂)ₙ-X group represented by R₃, X is preferably amino group, guanidino group, carbamoyl group (-CONH₂ group), pyrrole group, imidazole group, pyrazole group, or indole group, more preferably imidazole group. In the -(CH₂)ₙ-X group, n is preferably 1 or 2, more preferably 1.

Thus, the -(CH₂)ₙ-X group is preferably aminomethyl group, 2-aminoethyl group, 3-aminopropyl group, 4-aminobutyl group, carbamoylmethyl group, 2-carbamoylethyl group, 3-carbamoylbutyl group, 2-guanidinoethyl group, 3-guanidinobutyl group, pyrrolemethyl group, 4-imidazolemethyl group, pyrazolemethyl group, or 3-indolemethyl group, more preferably 4-aminobutyl group, carbamoylmethyl group, 2-carbamoylethyl group, 3-guanidinobutyl group, 4-imidazolemethyl group, or 3-indolemethyl group, still more preferably 4-imidazolemethyl group.

Particularly suitable lipidic peptide compounds of Formula (1) are the following compounds each being formed of a lipidic moiety and a peptide moiety (amino acid assembly), wherein the amino acid abbreviations are alanine (Ala), asparagine (Asn), glutamine (Gln), glycine (Gly), histidine (His), isoleucine (Ile), leucine (Leu), lysine (Lys), tryptophan (Trp), and valine (Val). Specific examples of the compounds include lauroyl-Gly-His, lauroyl-Gly-Gln, lauroyl-Gly-Asn, lauroyl-Gly-Trp, lauroyl-Gly-Lys, lauroyl-Ala-His, lauroyl-Ala-Gln, lauroyl-Ala-Asn, lauroyl-Ala-Trp, and lauroyl-Ala-Lys; myristoyl-Gly-His, myristoyl-Gly-Gln, myristoyl-Gly-Asn, myristoyl-Gly-Trp, myristoyl-Gly-Lys, myristoyl-Ala-His, myristoyl-Ala-Gln, myristoyl-Ala-Asn, myristoyl-Ala-Trp, and myristoyl-Ala-Lys; palmitoyl-Gly-His, palmitoyl-Gly-Gln, palmitoyl-Gly-Asn, palmitoyl-Gly-Trp, palmitoyl-Gly-Lys, palmitoyl-Ala-His, palmitoyl-Ala-Gln, palmitoyl-Ala-Asn, palmitoyl-Ala-Trp, and palmitoyl-Ala-Lys; and stearoyl-Gly-His, stearoyl-Gly-Gln, stearoyl-Gly-Asn, stearoyl-Gly-Trp, stearoyl-Gly-Lys, stearoyl-Ala-His, stearoyl-Ala-Gln, stearoyl-Ala-Asn, stearoyl-Ala-Trp, and stearoyl-Ala-Lys.

Most preferred are lauroyl-Gly-His, lauroyl-Ala-His, myristoyl-Gly-His, myristoyl-Ala-His, palmitoyl-Gly-His, palmitoyl-Ala-His, stearoyl-Gly-His, and stearoyl-Ala-His.

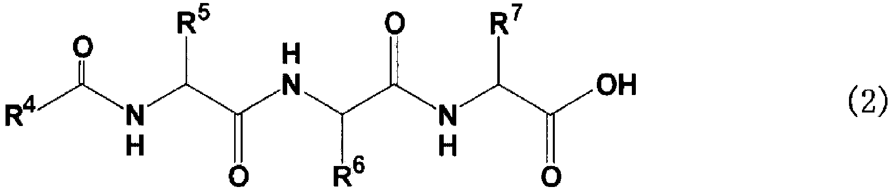

In Formula (2), R⁴ is a C₉₋₂₃ aliphatic group. Preferred specific examples of R⁴ include the same groups as those defined by R¹ above.

In Formula (2), R⁵ to R⁷ are each independently a hydrogen atom, a C₁₋₄ alkyl group having, if necessary a C₁ or C₂ branched chain, or a -(CH₂)ₙ-X group, and at least one of R⁵ to R⁷ is preferably a -(CH₂)ₙ-X group. In the -(CH₂)ₙ-X group, n is a number of 1 to 4, and X is an amino group, a guanidino group, a -CONH₂ group, or a 5-membered ring group or 6-membered ring group having, if necessary, one to three nitrogen atoms, or a fused heterocyclic group composed of the 5-membered ring and the 6-membered ring. Preferred specific examples of R⁵ to R⁷ include the same groups as those defined by R² and R³ above.

Suitable lipidic peptide compounds of Formula (2) are the following compounds each being formed of a lipidic moiety and a peptide moiety (amino acid assembly). Specific examples of the compounds include lauroyl-Gly-Gly-His, myristoyl-Gly-Gly-His, myristoyl-Gly-Gly-Gln, myristoyl-Gly-Gly-Asn, myristoyl-Gly-Gly-Trp, myristoyl-Gly-Gly-Lys, myristoyl-Gly-Ala-His, myristoyl-Gly-Ala-Gln, myristoyl-Gly-Ala-Asn, myristoyl-Gly-Ala-Trp, myristoyl-Gly-Ala-Lys, myristoyl-Ala-Gly-His, myristoyl-Ala-Gly-Gln, myristoyl-Ala-Gly-Asn, myristoyl-Ala-Gly-Trp, myristoyl-Ala-Gly-Lys, myristoyl-Gly-His-Gly, myristoyl-His-Gly-Gly, palmitoyl-Gly-Gly-His, palmitoyl-Gly-Gly-Gln, palmitoyl-Gly-Gly-Asn, palmitoyl-Gly-Gly-Trp, palmitoyl-Gly-Gly-Lys, palmitoyl-Gly-Ala-His, palmitoyl-Gly-Ala-Gln, palmitoyl-Gly-Ala-Asn, palmitoyl-Gly-Ala-Trp, palmitoyl-Gly-Ala-Lys, palmitoyl-Ala-Gly-His, palmitoyl-Ala-Gly-Gln, palmitoyl-Ala-Gly-Asn, palmitoyl-Ala-Gly-Trp, palmitoyl-Ala-Gly-Lys, palmitoyl-Gly-His-Gly, palmitoyl-His-Gly-Gly, and stearoyl-Gly-Gly-His.

Of these, most preferred are lauroyl-Gly-Gly-His, myristoyl-Gly-Gly-His, palmitoyl-Gly-Gly-His, palmitoyl-Gly-His-Gly, palmitoyl-His-Gly-Gly, and stearoyl-Gly-Gly-His.

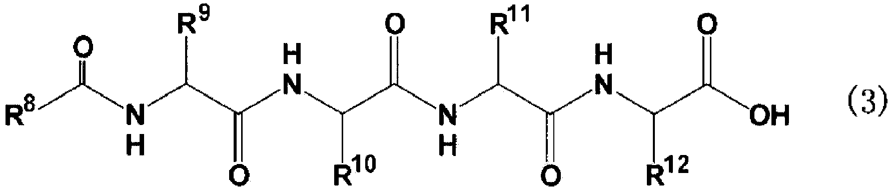

In Formula (3), R⁸ is a C₉₋₂₃ aliphatic group. Preferred specific examples of R⁸ include the same groups as those defined by R¹ above.

In Formula (3), R⁹ to R¹² are each independently a hydrogen atom, a C₁₋₄ alkyl group having, if necessary, a C₁ or C₂ branched chain, or a -(CH₂)ₙ-X group, and at least one of R⁹ to R¹² is preferably a -(CH₂)ₙ-X group. In the -(CH₂)ₙ-X group, n is a number of 1 to 4, and X is an amino group, a guanidino group, a -CONH₂ group, or a 5-membered ring group or 6-membered ring group having, if necessary, one to three nitrogen atoms, or a fused heterocyclic group composed of the 5-membered ring and the 6-membered ring. Preferred specific examples of R⁹ to R¹² include the same groups as those defined by R² and R³ above.

Thus, particularly suitable examples of the lipidic peptide compound of Formula (3) include lauroyl-Gly-Gly-Gly-His, myristoyl-Gly-Gly-Gly-His, palmitoyl-Gly-Gly-Gly-His, palmitoyl-Gly-Gly-His-Gly, palmitoyl-Gly-His-Gly-Gly, palmitoyl-His-Gly-Gly-Gly, and stearoyl-Gly-Gly-Gly-His.

In the present invention, the amount of the lipidic peptide compound contained in the anti-pollution material is, for example, 0.01 to 30% by mass, preferably 0.02 to 10% by mass, more preferably 0.03 to 5% by mass, still more preferably 0.05 to 1% by mass, relative to the total mass of the anti-pollution material.

The lipidic peptide compound used in the present invention is composed of at least one of compounds (lipidic peptides) of Formulae (1) to (3) or pharmaceutically usable salts of the compounds. These compounds may be used alone or in combination of two or more species.

### [Additional Ingredient]

The anti-pollution material of the present invention may contain, besides the aforementioned lipidic peptide compound, water, an alcohol, a polyhydric alcohol, or a mixed solution thereof.

Examples of the aforementioned water include clean water, purified water, hard water, soft water, natural water, deep-sea water, electrolytic alkaline ionized water, electrolytic acidic ionized water, ionized water, and cluster water.

The aforementioned alcohol refers to a monohydric alcohol. Examples of the monohydric alcohol include C₁₋₆ alcohols that dissolve in water in any proportion, such as methanol, ethanol, 2-propanol, and i-butanol; and higher alcohols, such as oleyl alcohol and phenoxy alcohol.

The aforementioned polyhydric alcohol refers to a di- or more-valent alcohol. Examples of the polyhydric alcohol include propylene glycol, 1,3-butanediol, 2-ethyl-1,3-hexanediol, glycerin, isopentyldiol, ethylhexanediol, erythrulose, ozonated glycerin, caprylyl glycol, glycol, (C15-18) glycol, (C20-30) glycol, diethylene glycol, diglycerin, dithiaoctanediol, DPG, thioglycerin, 1,10-decanediol, decylene glycol, triethylene glycol, trimethylhydroxymethylcyclohexanol, phytantriol, phenoxypropanediol, 1,2-butanediol, 2,3-butanediol, butylethylpropanediol, 1,2-hexanediol, hexylene glycol, pentylene glycol, methylpropanediol, menthanediol, lauryl glycol, and polypropylene glycol.

In the present invention, when the anti-pollution material contains a polyhydric alcohol, the amount of the polyhydric alcohol may be, for example, 1% by mass to 60% by mass, preferably 1% by mass to 30% by mass, relative to the total mass of the anti-pollution material.

In the present invention, when the anti-pollution material contains a polyhydric alcohol, a single polyhydric alcohol may be used, or two or more polyhydric alcohols may be used in combination.

### [Other Additives]

If necessary, the anti-pollution material of the present invention may contain additives generally usable as additives for cosmetic products, quasi-drugs, and pharmaceutical products.

Examples of additive ingredients such as physiologically active substances and functional substances contained in external formulations for skin (e.g., cosmetic products, quasi-drugs, or pharmaceutical products) include pigments, oily bases, humectants, texture improvers, surfactants other than those described above, polymers, thickeners, gelators, solvents, antioxidants, reducing agents, oxidizers, preservatives, antimicrobial agents, antiseptics, chelating agents, pH adjusters, acids, alkalis, powders, inorganic salts, ultraviolet absorbers, whitening agents, vitamins and derivatives thereof, hair growth-promoting agents, blood circulation promoters, stimulants, hormones, anti-wrinkle agents, anti-aging agents, firming agents, cooling agents, warming agents, wound-healing promoters, abirritants, analgesics, cell activators, plant/animal/microbial extracts, antipruritics, exfoliates/keratolytic agents, antiperspirants, algefacients, astringents, enzymes, nucleic acids, perfumes, colors, coloring agents, dyes, antiphlogistics, anti-inflammatory agents, anti-asthmatic agents, anti-chronic obstructive pulmonary disease agents, anti-allergic agents, immunomodulators, anti-infective agents, and antifungal agents.

The amount of such an additive contained in the anti-pollution material may vary depending on the type of the additive. The amount of the additive may be, for example, 0.001% by mass to 20% by mass or about 0.01% by mass to 10% by mass, relative to the total mass of the anti-pollution material.

### [Pigment]

Preferred examples of pigments include inorganic white pigments, such as titanium dioxide and zinc oxide; inorganic red pigments, such as iron oxide (red iron oxide) and iron titanate; inorganic brown pigments, such as γ-iron oxide; inorganic yellow pigments, such as yellow iron oxide and ocher; inorganic black pigments, such as black iron oxide and low-order titanium oxide; inorganic violet pigments, such as mango violet and cobalt violet; inorganic green pigments, such as chromium oxide, chromium hydroxide, and cobalt titanate; inorganic blue pigments, such as ultramarine and Prussian blue; pearl pigments, such as titanium oxide-coated mica, titanium oxide-coated bismuth oxychloride, titanium oxide-coated talc, colored titanium oxide-coated mica, bismuth oxychloride, and argentine; extender pigments, such as talc, sericite, mica, kaolin, calcium carbonate, magnesium carbonate, silicic anhydride, barium sulfate, and aluminum hydroxide; metal powder pigments, such as aluminum powder, copper powder, and gold; surface-treated inorganic and metal powder pigments; organic pigments, such as zirconium, barium, or aluminum lake; and surface-treated organic pigments.

Preferred examples of oily bases include higher (polyhydric) alcohols, such as oleyl alcohol, jojoba alcohol, chimyl alcohol, selachyl alcohol, batyl alcohol, hexyldecanol, isostearyl alcohol, 2-octyldodecanol, and dimer diols; aralkyl alcohols and derivatives thereof, such as benzyl alcohol; stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, long-chain branched fatty acids, dimer acids, and hydrogenated dimer acids; hydrocarbons, such as liquid paraffin (mineral oil), heavy liquid isoparaffin, light liquid isoparaffin, α-olefin oligomers, polyisobutene, hydrogenated polyisobutene, polybutene, squalane, olive-derived squalane, squalene, vaseline, and solid paraffin; waxes, such as candelilla wax, carnauba wax, rice wax, Japan wax, beeswax, montan wax, ozokerite, ceresin, paraffin wax, microcrystalline wax, petrolatum, Fischer-Tropsch wax, polyethylene wax, and ethylene-propylene copolymers; vegetable oils and fats, such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, hydrogenated jojoba oil, grape seed oil, apricot oil (apricot kernel oil), and camellia oil; animal oils and fats, such as beef tallow, milk fat, horse fat, egg-yolk oil, mink oil, and turtle oil; animal waxes, such as spermaceti, lanolin, and orange roughy oil; lanolins, such as liquid lanolin, reduced lanolin, adsorption-purified lanolin, acetylated lanolin, acetylated liquid lanolin, hydroxylated lanolin, polyoxyethylene lanolin, lanolin fatty acids, hard lanolin fatty acids, lanolin alcohol, acetylated lanolin alcohol, and acetylated (cetyl/lanolyl) ester; sterols, such as cholesterol, dihydrocholesterol, lanosterol, dihydrolanosterol, phytosterol, and cholic acid; sapogenins; saponins; sterol esters, such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroylsarcosinate, cholesteryl 12-hydroxystearate, cholesteryl macadamiate, phytosteryl macadamiate, phytosteryl isostearate, soft lanolin fatty acid cholesteryl esters, hard lanolin fatty acid cholesteryl esters, long-chain branched fatty acid cholesteryl esters, and long-chain α-hydroxy fatty acid cholesteryl esters; lipidic complexes, such as phospholipid-cholesterol complexes and phospholipid-phytosterol complexes; monohydric alcohol esters of carboxylic acids, such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters, such as cetyl lactate, diisostearyl malate, and hydrogenated castor oil monoisostearate; polyhydric alcohol esters of fatty acids, such as glyceryl trioctanoate (glyceryl tri-2-ethylhexanoate), glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl ester, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), 3-methyl-1,5-pentanediol dineopentanoate, and 2,4-diethyl-1,5-pentanediol dineopentanoate; dimer acid or dimer diol derivatives, such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate, and hydroxyalkyl dimer dilinoleyl ether; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamide (cocamide methyl MEA); silicones, such as dimethicone (dimethylpolysiloxane), highly polymerized dimethicone (highly polymerized dimethylpolysiloxane), cyclomethicone (cyclic dimethylsiloxane, decamethylcyclopentasiloxane (which may also be referred to simply as "cyclopentasiloxane")), phenyl trimethicone, diphenyl dimethicone, phenyl dimethicone, stearoxypropyldimethylamine, (aminoethylaminopropyl methicone/dimethicone) copolymers, dimethiconol, dimethiconol crosspolymers, silicone resin, silicone rubber, amino-modified silicones such as aminopropyl dimethicone and amodimethicone, cation-modified silicones, polyether-modified silicones such as dimethicone copolyols, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine oils, such as perfluorodecane, perfluorooctane, and perfluoropolyether.

Preferred examples of humectants and texture improvers include polyols and polymers thereof, such as glycerin, trimethylolpropane, pentaerythritol, hexylene glycol, diglycerin, polyglycerin, diethylene glycol, dipropylene glycol, polypropylene glycol, and ethylene glycol-propylene glycol copolymers; glycol alkyl ethers, such as diethylene glycol monoethyl ether (ethoxydiglycol), ethylene glycol monoethyl ether, ethylene glycol monobutyl ether, and diethylene glycol dibutyl ether; water-soluble esters, such as polyglyceryl-10 (eicosanedioate/tetradecanedioate) and polyglyceryl-10 tetradecanedioate; sugar alcohols, such as sorbitol, xylitol, erythritol, mannitol, and maltitol; saccharides and derivatives thereof, such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and modified cyclodextrins such as maltosyl cyclodextrin and hydroxyalkyl cyclodextrin), β-glucan, chitin, chitosan, heparin and derivatives thereof, pectin, arabinogalactan, dextrin, dextran, glycogen, ethyl glucoside, and glucosylethyl methacrylate polymer or copolymer; hyaluronic acid and sodium hyaluronate; sodium chondroitin sulfate; mucoitin sulfate, charonin sulfate, keratosulfate, and dermatan sulfate; Tremella fuciformis extract and Tremella fuciformis polysaccharides; fucoidan; tuberose polysaccharides or naturally occurring polysaccharides; organic acids such as citric acid, tartaric acid, and lactic acid, and salts thereof; urea and derivatives thereof; 2-pyrrolidone-5-carboxylic acid and salts thereof, such as sodium salt; amino acids, such as betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cysteine, cystine, methionine, leucine, isoleucine, valine, tryptophan, histidine, and taurine, and salts thereof; protein peptides and derivatives thereof, such as collagen, fish-derived collagen, atelocollagen, gelatin, elastin, peptides derived from degraded collagen, hydrolyzed collagen, hydroxypropylammonium chloride hydrolyzed collagen, peptides derived from degraded elastin, peptides derived from degraded keratin, hydrolyzed keratin, peptides derived from degraded conchiolin, hydrolyzed conchiolin, peptides derived from degraded silk protein, hydrolyzed silk, sodium lauroyl hydrolyzed silk, peptides derived from degraded soy protein, peptides derived from degraded wheat protein, hydrolyzed wheat protein, peptides derived from degraded casein, and acylated peptides; acylated peptides, such as palmitoyl oligopeptide, palmitoyl pentapeptide, and palmitoyl tetrapeptide; silylated peptides; lactic acid bacteria culture, yeast extracts, eggshell membrane protein, bovine submaxillary mucin, hypotaurine, sesame lignan glycosides, glutathione, albumin, and whey; choline chloride and phosphorylcholine; animal and plant extract ingredients, such as placenta extract, elastin, collagen, aloe extract, Hamamelis virginiana water, Luffa cylindrica water, Chamomilla recutita extract, licorice extract, comfrey extract, silk extract, Rosa roxburghii extract, Achillea millefolium extract, Eucalyptus globulus extract, and melilot extract; and ceramides, such as natural ceramides (types 1, 2, 3, 4, 5, and 6), hydroxyceramide, pseudoceramide, sphingoglycolipid, ceramide-containing extract, and glucosylceramide-containing extract.

Preferred examples of surfactants include anionic surfactants, nonionic surfactants, cationic surfactants, amphoteric surfactants, and polymer surfactants. Preferred examples of these surfactants are as follows. Preferred examples of anionic surfactants include fatty acid salts, such as potassium laurate and potassium myristate; alkyl sulfates, such as sodium lauryl sulfate, triethanolamine lauryl sulfate, and ammonium lauryl sulfate; polyoxyethylene alkyl sulfates, such as sodium laureth sulfate and triethanolamine laureth sulfate; acyl N-methylamino acid salts, such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate, and sodium lauroyl glutamate methyl alaninate; acyl amino acid salts, such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alaninate; polyoxyethylene alkyl ether acetates, such as sodium laureth acetate; succinates, such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates, such as sodium hydrogenated coconut oil fatty acid glycerin sulfates; alkylbenzene polyoxyethylene sulfates; olefin sulfonates. such as sodium α-olefin sulfonates; alkyl sulfosuccinates, such as disodium lauryl sulfosuccinate and sodium dioctyl sulfosuccinate; alkyl ether sulfosuccinates, such as disodium laureth sulfosuccinate, sodium monolauroyl monoethanolamide polyoxyethylene sulfosuccinate, and sodium lauryl polypropylene glycol sulfosuccinate; alkylbenzene sulfonates, such as sodium tetradecylbenzene sulfonate and triethanolamine tetradecylbenzene sulfonate; alkyl naphthalene sulfonates; alkane sulfonates; α-sulfofatty acid methyl ester salts; acyl isethionates; alkyl glycidyl ether sulfonates; alkyl sulfoacetates; alkyl ether phosphates, such as sodium laureth phosphate, sodium dilaureth phosphate, sodium trilaureth phosphate, and sodium monooreth phosphate; alkyl phosphates, such as potassium lauryl phosphate; sodium caseinate; alkyl aryl ether phosphates; fatty acid amide ether phosphates; phospholipids, such as phosphatidylglycerol, phosphatidylinositol, and phosphatidic acid; and silicone anionic surfactants, such as carboxylic acid-modified silicones, phosphoric acid-modified silicones, and sulfuric acid-modified silicones. Preferred examples of nonionic surfactants include polyoxyethylene alkyl ethers with various numbers of polyoxyethylene units, such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyldodecyl ethers); polyoxyethylene alkyl phenyl ethers; castor oil derivatives and hydrogenated castor oil derivatives, such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester, and polyoxyethylene hydrogenated castor oil maleate; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers, such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols, such as PPG-9 diglyceryl; glycerin fatty acid partial esters, such as glyceryl stearate, glyceryl isostearate, glyceryl palmitate, glyceryl myristate, glyceryl oleate, glyceryl cocoate, glycerin mono-cottonseed oil fatty acid esters, glycerin monoerucate, glycerin sesquioleate, glycerin α,α'-oleate pyroglutamate, and glycerin monostearate malate; polyglycerin fatty acid esters, such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono-fatty acid esters, such as ethylene glycol monostearate; propylene glycol mono-fatty acid esters, such as propylene glycol monostearate; pentaerythritol fatty acid partial esters; sorbitol fatty acid partial esters; maltitol fatty acid partial esters; maltitol ethers; sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; sugar derivative partial esters, such as sucrose fatty acid esters, methyl glucoside fatty acid esters, and trehalose undecylenoate; alkyl glucosides, such as caprylyl glucoside; alkyl polyglycosides; lanolin alcohol; reduced lanolin; polyoxyethylene fatty acid mono- and di-esters, such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters, such as polyoxyethylene monooleates, for example, polyoxyethylene glycerin monostearate, polyoxyethylene glycerin monoisostearate, and polyoxyethylene glycerin triisostearate; polyoxyethylene sorbitan fatty acid esters, such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters, such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene animal and vegetable fats and oils, such as polyoxyethylene sorbitol beeswax; alkyl glyceryl ethers, such as isostearyl glyceryl ether, chimyl alcohol, selachyl alcohol, and batyl alcohol; polyhydric alcohol alkyl ethers; polyoxyethylene alkylamines; tetrapolyoxyethylene/tetrapolyoxypropylene-ethylenediamine condensates; natural surfactants, such as saponin and sophorolipid; polyoxyethylene fatty acid amides; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamides (cocamide methyl MEA); alkyl dimethylamine oxides, such as lauramine oxide, cocamine oxide, stearamine oxide, and behenamine oxide; alkyl ethoxydimethylamine oxides; polyoxyethylene alkyl mercaptans; and silicone nonionic surfactants, for example, polyether-modified silicones such as dimethicone copolyols, polysiloxane-oxyalkylene copolymers, polyglycerin-modified silicones, and sugar-modified silicones. Preferred examples of cationic surfactants include alkyl trimethylammonium chlorides, such as behentrimonium chloride, steartrimonium chloride, cetrimonium chloride, and lauryltrimonium chloride; alkyl trimethylammonium bromides, such as steartrimonium bromide; dialkyl dimethylammonium chlorides, such as distearyldimonium chloride and dicocodimonium chloride; fatty acid amide amines, such as stearamidopropyl dimethylamine and stearamidoethyl diethylamine, and salts thereof; alkyl ether amines, such as stearoxypropyldimethylamine and salts or quaternary salts thereof; fatty acid amide quaternary ammonium salts, such as long-chain branched fatty acid (12 to 31) aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidium salts; alkyl ether amine ammonium salts; alkyl trialkylene glycol ammonium salts; benzalkonium salts; benzethonium salts; pyridinium salts, such as cetylpyridinium chloride; imidazolinium salts; alkyl isoquinolinium salts; dialkyl morpholinium salts; polyamine fatty acid derivatives; and silicone cationic surfactants, such as amino-modified silicones such as aminopropyl dimethicone and amodimethicone, cation-modified silicones, cation-modified and polyether-modified silicones, and amino-modified and polyether-modified silicones. Preferred examples of amphoteric surfactants include N-alkyl-N,N-dimethylamino acid betaines, such as lauryl betaine (lauryl dimethylaminoacetic acid betaine); fatty acid amidoalkyl-N,N-dimethylamino acid betaines, such as cocamidopropyl betaine and lauramidopropyl betaine; imidazoline betaines, such as sodium cocoamphoacetate and sodium lauroamphoacetate; alkyl sulfobetaines, such as alkyl dimethyltaurine; betaine sulfates, such as alkyl dimethylaminoethanol sulfate; betaine phosphates, such as alkyl dimethylaminoethanol phosphates; phospholipids, such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid, and hydroxylated lecithin; and silicone amphoteric surfactants. Preferred examples of polymer surfactants include polyvinyl alcohol, sodium alginate, starch derivatives, tragacanth gum, and acrylic acid-alkyl methacrylate copolymers; and various silicone surfactants.

Preferred examples of polymers, thickeners, and gelators include guar gum, locust bean gum, quince seed, carrageenan, galactan, gum arabic, tara gum, tamarind, furcellaran, karaya gum, Abelmoschus manihot, cara gum, tragacanth gum, pectin, pectic acid and salts thereof, such as sodium salt, alginic acid and salts thereof, such as sodium salt, and mannan; starches, such as rice starch, corn starch, potato starch, and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; celluloses and derivatives thereof, such as methyl cellulose, ethyl cellulose, hydroxyethyl cellulose, hydroxypropyl cellulose, hydroxypropyl methylcellulose, carboxymethyl cellulose and salts thereof, such as sodium salt, methylhydroxypropyl cellulose, sodium cellulose sulfate, dialkyldimethylammonium cellulose sulfate, crystalline cellulose, and cellulose powder; starch derivatives, such as soluble starch, starch polymers such as carboxymethyl starch, methylhydroxypropyl starch, and methyl starch, starch hydroxypropyltrimonium chloride, and aluminum corn starch octenylsuccinate; alginic acid derivatives, such as sodium alginate and propylene glycol alginate; polyvinylpyrrolidone (PVP), polyvinyl alcohol (PVA), vinylpyrrolidone-vinyl alcohol copolymers, and polyvinyl methyl ether; polyethylene glycol, polypropylene glycol, and polyoxyethylene-polyoxypropylene copolymers; amphoteric methacrylic acid ester copolymers, such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers, and (alkyl acrylate/diacetone acrylamide) copolymers AMP; partially saponified polyvinyl acetate and maleic acid copolymers; vinylpyrrolidone-dialkylaminoalkyl methacrylate copolymers; acrylic resin alkanolamine; polyester and water-dispersible polyester; polyacrylamide; copolymers of polyacrylic esters such as polyethyl acrylate, carboxy vinyl polymers, polyacrylic acid and salts thereof, such as sodium salt, and acrylic acid-methacrylic acid ester copolymers; acrylic acid-alkyl methacrylate copolymers; cationized celluloses such as polyquaternium-10, diallyldimethylammonium chloride-acrylamide copolymers, such as polyquaternium-7, acrylic acid-diallyldimethylammonium chloride copolymers, such as polyquaternium-22, acrylic acid-diallyldimethylammonium chloride-acrylamide copolymers, such as polyquaternium-39, acrylic acid-cationized methacrylic acid ester copolymers, acrylic acid-cationized methacrylic acid amide copolymers, acrylic acid-methyl acrylate-methacrylamidopropyltrimethylammonium chloride copolymers, such as polyquaternium-47, and methacryloyl chloride choline ester polymers; cationized polysaccharides, such as cationized oligosaccharides, cationized dextran, and guar hydroxypropyltrimonium chloride; polyethyleneimine; cationic polymers; copolymers of 2-methacryloyloxyethyl phosphorylcholine and butyl methacrylate, such as polyquaternium-51; polymer emulsions, such as acrylic resin emulsions, polyethyl acrylate emulsions, polyacrylalkyl ester emulsions, polyvinyl acetate resin emulsions, natural rubber latex, and synthetic latex; nitrocellulose; polyurethanes and various copolymers thereof; various silicones; various silicone copolymers, such as acrylic-silicone graft copolymers; various fluorine polymers; 12-hydroxystearic acid and salts thereof; dextrin fatty acid esters, such as dextrin palmitate and dextrin myristate; and silicic anhydride, fumed silica (silicic anhydride ultrafine particles), magnesium aluminum silicate, magnesium sodium silicate, metallic soaps, dialkyl phosphate metal salts, bentonite, hectorite, organic-modified clay minerals, sucrose fatty acid esters, and fructooligosaccharide fatty acid esters. Among the aforementioned examples, preferred are cellulose and derivatives thereof, alginic acid and salts thereof, polyvinyl alcohol, hyaluronic acid and salts thereof, or collagen.

Preferred examples of solvents include lower alcohols, such as ethanol, 2-propanol (isopropyl alcohol), butanol, and isobutyl alcohol; glycols, such as propylene glycol, diethylene glycol, dipropylene glycol, and isopentyldiol; glycol ethers, such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters, such as ethylene glycol monoethyl ether acetate, diethylene glycol monoethyl ether acetate, and propylene glycol monoethyl ether acetate; glycol esters, such as diethoxyethyl succinate and ethylene glycol disuccinate; benzyl alcohol, benzyloxyethanol, propylene carbonate, dialkyl carbonate, acetone, ethyl acetate, and N-methylpyrrolidone; and toluene.

Preferred examples of antioxidants include tocopherol (vitamin E) and tocopherol derivatives, such as tocopherol acetate; BHT and BHA; gallic acid derivatives, such as propyl gallate; vitamin C (ascorbic acid) and/or derivatives thereof; erythorbic acid and derivatives thereof; sulfites, such as sodium sulfite; hydrogen sulfites, such as sodium hydrogen sulfite; thiosulfates, such as sodium thiosulfate; metabisulfites; thiotaurine and hypotaurine; and thioglycerol, thiourea, thioglycolic acid, and cysteine hydrochloride.

Preferred examples of reducing agents include thioglycolic acid, cysteine, and cysteamine.

Preferred examples of oxidizers include hydrogen peroxide solution, ammonium persulfate, sodium bromate, and percarbonic acid.

Preferred examples of preservatives, antimicrobial agents, and antiseptics include hydroxybenzoic acids and salts or esters thereof, such as methylparaben, ethylparaben, propylparaben, and butylparaben; salicylic acid; sodium benzoate; phenoxyethanol; isothiazolinone derivatives, such as methylchloroisothiazolinone and methylisothiazolinone; imidazolinium urea; dehydroacetic acid and salts thereof; phenols; halogenated bisphenols, such as triclosan, acid amides thereof, and quaternary ammonium salts thereof; trichlorocarbanide, zinc pyrithione, benzalkonium chloride, benzethonium chloride, sorbic acid, chlorhexidine, chlorhexidine gluconate, halocarban, hexachlorophene, and hinokitiol; other phenols, such as phenol, isopropylphenol, cresol, thymol, parachlorohenol, phenylphenol, and sodium phenylphenate; phenyl ethyl alcohol, photosensitizers, antibacterial zeolite, and silver ions.

Preferred examples of chelating agents include edetates (ethylenediamine tetraacetates), such as EDTA, EDTA 2Na, EDTA 3Na, and EDTA 4Na; hydroxyethylethylenediamine triacetates, such as HEDTA 3Na; pentetates (diethylenetriamine pentaacetate); phytic acid; phosphonic acids, such as etidronic acid, and salts thereof, such as sodium salt; polyamino acids, such as polyaspartic acid and polyglutamic acid; sodium polyphosphate, sodium metaphosphate, and phosphoric acid; and sodium citrate, citric acid, alanine, dihydroxyethylglycine, gluconic acid, ascorbic acid, succinic acid, and tartaric acid.

Preferred examples of pH adjusters, acids, and alkalis include ascorbic acid, citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, aqueous ammonia, guanidine carbonate, and ammonium carbonate.

Preferred examples of powders include inorganic powders having various sizes and shapes, such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, zeolite, barium sulfate, baked calcium sulfate, calcium phosphate such as tricalcium phosphate, fluorapatite, hydroxyapatite, ceramic powder, bentonite, smectite, clay, mud, metallic soaps (e.g., zinc myristate, calcium palmitate, and aluminum stearate), calcium carbonate, red iron oxide, yellow iron oxide, black iron oxide, ultramarine, Prussian blue, carbon black, titanium oxide, titanium oxide fine particles and titanium oxide ultrafine particles, zinc oxide, zinc oxide fine particles and zinc oxide ultrafine particles, alumina, silica, fumed silica (silicic anhydride ultrafine particles), titanated mica, argentine, boron nitride, photochromic pigments, synthetic fluorophlogopite, particulate composite powders, gold, silver, platinum, and aluminum; inorganic powders, such as hydrophobic or hydrophilic powders prepared by treating the aforementioned powders with various surface-treating agents such as silicones (e.g., hydrogen silicone and cyclic hydrogen silicone) or other silanes or titanium coupling agents; organic powders having various sizes and shapes, such as starch, cellulose, nylon powder, polyethylene powder, polymethyl methacrylate powder, polystyrene powder, styrene-acrylic acid copolymer resin powder, polyester powder, benzoguanamine resin powder, polyethylene terephthalate-polymethyl methacrylate laminated powder, polyethylene terephthalate-aluminum-epoxy laminated powder, urethane powder, silicone powder, and Teflon (registered trademark) powder; surface-treated organic powders; and organic-inorganic composite powders.

Preferred examples of inorganic salts include sodium chloride-containing salts, such as common salt, regular salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; and sodium phosphates, such as mono-, di-, and tri-sodium phosphates, potassium phosphates, calcium phosphates, and magnesium phosphates.

Preferred examples of ultraviolet absorbers include benzoic acid ultraviolet absorbers, such as p-aminobenzoic acid, p-aminobenzoic acid monoglycerin ester, N,N-dipropoxy-p-aminobenzoic acid ethyl ester, N,N-diethoxy-p-aminobenzoic acid ethyl ester, N,N-dimethyl-p-aminobenzoic acid ethyl ester, N,N-dimethyl-p-aminobenzoic acid butyl ester, and N,N-dimethyl-p-aminobenzoic acid methyl ester; anthranilic acid ultraviolet absorbers, such as homomenthyl-N-acetylanthranilate; salicylic acid ultraviolet absorbers, such as salicylic acid and sodium salt thereof, amyl salicylate, menthyl salicylate, homomenthyl salicylate, octyl salicylate, phenyl salicylate, benzyl salicylate, and p-isopropanolphenyl salicylate; cinnamic acid ultraviolet absorbers, such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate (octyl p-methoxycinnamate), 2-ethoxyethyl-p-methoxycinnamate (cinoxate), cyclohexyl-p-methoxycinnamate, ethyl-α-cyano-β-phenylcinnamate, 2-ethylhexyl-α-cyano-β-phenylcinnamate (octocrylene), glyceryl mono-2-ethylhexanoyl-dip-methoxycinnamate, ferulic acid, and derivatives thereof; benzophenone ultraviolet absorbers, such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor and 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives, such as 4-t-butylmethoxydibenzoylmethane; octyl triazone; urocanic acid and urocanic acid derivatives, such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives, such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of whitening agents include hydroquinone glycosides, such as arbutin and α-arbutin, and esters thereof; ascorbic acid, and ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbic acid alkyl ethers, such as ascorbic acid ethyl ether, ascorbic acid glucosides, such as ascorbic acid 2-glucoside and fatty acid esters thereof, ascorbyl sulfate, and tocopheryl ascorbyl phosphate; kojic acid, ellagic acid, tranexamic acid and derivatives thereof; ferulic acid and derivatives thereof; placenta extract, glutathione, oryzanol, butylresorecinol, and plant extracts, such as oil-soluble chamomilla extract, oil-soluble licorice extract, Tamarix chinensis extract, and Saxifraga stolonifera extract.

Preferred examples of vitamins and derivatives thereof include forms of vitamin A, such as retinol, retinol acetate, and retinol palmitate; forms of vitamin B, such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acid products, nicotinic acid products, such as nicotinamide and benzyl nicotinate, and choline products; forms of vitamin C, such as ascorbic acid and salts thereof, such as sodium salt; vitamin D; forms of vitamin E, such as α-, β-, γ-, and δ-tocopherols; other vitamins, such as pantothenic acid and biotin; ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbyl stearate, ascorbyl palmitate, and ascorbyl dipalmitate, ascorbic acid alkyl ethers, such as ascorbic acid ethyl ether, ascorbic acid glucosides, such as ascorbic acid-2-glucoside and fatty acid esters thereof, and tocopheryl ascorbyl phosphate; vitamin derivatives, for example, tocopherol derivatives, such as tocopherol nicotinate, tocopherol acetate, tocopherol linoleate, tocopherol ferulate, and tocopherol phosphate, tocotrienol, and other various vitamin derivatives.

Preferred examples of hair growth-promoting agents, blood circulation promoters, and stimulants include plant extracts and tinctures, such as swertia herb extract, capsicum tincture, ginger tincture, ginger extract, and cantharis tincture; capsaicin, nonylic acid vanillylamide, zingerone, ichthammol, tannic acid, borneol, cyclandelate, cinnarizine, tolazoline, acetylcholine, verapamil, cepharanthine, γ-oryzanol, vitamin E and derivatives thereof, such as tocopherol nicotinate and tocopherol acetate, γ-oryzanol, nicotinic acid and derivatives thereof, such as nicotinamide, benzyl nicotinate, inositol hexanicotinate, and nicotinic alcohol, allantoin, photosensitizer 301, photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone, and prednisone.

Preferred examples of other medicinal agents, such as anti-wrinkle agents, anti-aging agents, firming agents, cooling agents, warming agents, wound-healing promoters, abirritants, analgesics, and cell activators include retinol products, retinoic acid products, and tocopheryl retinoate; lactic acid, glycolic acid, gluconic acid, fruit acid, salicylic acid, and derivatives thereof, such as glycosides and esters, and α- or β-hydroxy acids and derivatives thereof, such as hydroxycapric acid, long-chain α-hydroxy fatty acids, and long-chain α-hydroxy fatty acid cholesteryl esters; γ-aminobutyric acid and γ-amino-P-hydroxybutyric acid; carnitine; carnocin; creatine; ceramides and sphingosines; caffeine, xanthine, and derivatives thereof; antioxidants and active oxygen scavengers, such as coenzyme Q10, carotene, lycopene, astaxanthin, lutein, α-lipoic acid, platinum nanocolloid, and fullerenes; catechins; flavones, such as quercetin; isoflavones; gallic acid and sugar ester derivatives thereof; polyphenols, such as tannin, sesamin, proanthocyanidin, chlorogenic acid, and apple polyphenols; rutin and derivatives thereof, such as glycosides; hesperidin and derivatives thereof, such as glycosides; lignan glycosides; licorice extract-related substances, such as glabridin, glabrene, liquiritin, and isoliquiritin; lactoferrin; shogaol and gingerol; perfume substances, such as menthol and cedrol, and derivatives thereof; capsaicin, vanillin, and derivatives thereof; insect repellents, such as diethyltoluamide; and complexes of physiologically active substances and cyclodextrins.

Preferred examples of plant, animal, and microbial extracts include iris extract, Angelica keiskei extract, Thujopsis dolabrata extract, asparagus extract, avocado extract, Hydrangea serrata leaf extract, almond extract, Althea officinalis root extract, Arnica montana extract, aloe extract, apricot extract, apricot kernel extract, ginkgo extract, Artemisia capillaris flower extract, fennel fruit extract, turmeric root extract, oolong tea extract, uva-ursi extract, rose fruit extract, Echinacea angustifolia leaf extract, Isodonis japonicus extract, Scutellaria root extract, Phellodendron bark extract, Coptis rhizome extract, barley extract, Panax ginseng extract, Hypericum perforatum extract, Lamium album extract, Ononis spinosa extract, Nasturtium officinale extract, orange extract, dried sea water residues, seaweed extract, Japanese persimmon leaf extract, Pyracantha fortuneana fruit extract, hydrolyzed elastin, hydrolyzed wheat powder, hydrolyzed silk, Pueraria root extract, Chamomilla recutita extract, oil-soluble Chamomilla recutita extract, carrot extract, Artemisia capillaris extract, Avena fatua extract, Hibiscus sabdariffa extract, licorice extract, oil-soluble licorice extract, kiwi fruit extract, kiou extract, Auricularia auricula-judae extract, Cinchona bark extract, cucumber extract, Paulownia tomentosa leaf extract, guanosine, guava extract, Sophora root extract, Gardenia jasminoides extract, Sasa veitchii extract, Sophora flavescens extract, walnut extract, chestnut extract, grapefruit extract, Clematis vitalba extract, black rice extract, black sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, Asiasarum root extract, Bupleurum root extract, umbilical cord extract, saffron extract, salvia extract, Saponaria officinalis extract, bamboo grass extract, Crataegus cuneata fruit extract, Bombyx mori excrementum extract, Zanthoxylum piperitum peel extract, shiitake mushroom extract, Rehmannia glutinosa root extract, Lithospermum root extract, Perilla frutescens extract, Tilia japonica extract, Filipendula multijuga extract, jatoba extract, peony root extract, ginger extract, Acorus calamus root extract, Betula alba extract, Tremella fuciformis extract, Equisetum arvense extract, stevia extract, stevia fermentation product, Tamarix chinensis extract, Hedera helix extract, Crataegus oxycantha extract, Sambucus nigra extract, Achillea millefolium extract, Mentha piperita extract, sage extract, mallow extract, Cnidium rhizome extract, swertia herb extract, mulberry bark extract, rhubarb extract, soybean extract, jujubi extract, thyme extract, dandelion extract, lichens extract, tea extract, clove extract, Imperata cylindrica extract, Citrus unshiu peel extract, tea tree oil, Rubus suavissimus extract, capsicum extract, Angelica acutiloba root extract, Calendula officinalis extract, peach kernel extract, bitter orange peel extract, Houttuynia cordata extract, tomato extract, natto extract, carrot extract, garlic extract, Rosa canina fruit extract, hibiscus extract, Ophiopogon tuber extract, lotus extract, parsley extract, birch extract, honey, Hamamelis virginiana extract, Parietaria officinalis extract, Rabdosia japonica extract, bisabolol, cypress extract, Bifidobacterium extract, Eriobotiya japonica extract, Tussilago farfara extract, Japanese butterbur flower-bud extract, Poria cocos sclerotium extract, butcher's broom extract, grape extract, grape seed extract, propolis, Luffa cylindrica extract, safflower extract, peppermint extract, Tilia miqueliaria extract, Paeonia suffruticosa extract, hop extract, Rosa rugosa extract, pine extract, Aesculus hippocastanum extract, Lysichiton camtschatcense extract, Sapindus mukurossi extract, Melissa officinalis extract, Nemacystus decipiens extract, peach extract, cornflower extract, Eucalyptus globulus extract, Saxifraga stolonifera extract, Citrus junos extract, lily extract, coix seed extract, Artemisia princeps extract, lavender extract, green tea extract, egg shell membrane extract, apple extract, rooibos tea extract, Litchi chinensis extract, lettuce extract, lemon extract, Forsythia fruit extract, Astragalus sinicus extract, rose extract, rosemary extract, Anthemis nobilis extract, royal jelly extract, and Sanguisorba officinalis extract.

Examples of antipruritics include diphenhydramine hydrochloride, chlorpheniramine maleate, camphor, and substance-P inhibitors.

Examples of exfoliates/keratolytic agents include salicylic acid, sulfur, resorcin, selenium sulfide, and pyridoxine.

Examples of antiperspirants include aluminum chlorohydrate, aluminum chloride, zinc oxide, and zinc p-phenolsulfonate.

Examples of algefacients include menthol and methyl salicylate.

Examples of astringents include citric acid, tartaric acid, lactic acid, aluminum potassium sulfate, and tannic acid.

Examples of enzymes include superoxide dismutase, catalase, lysozyme chloride, lipase, papain, pancreatin, and protease.

Preferred examples of nucleic acids include ribonucleic acids and salts thereof, deoxyribonucleic acids and salts thereof, and adenosine triphosphate disodium.

Preferred examples of perfumes include synthetic and natural perfumes and various compound perfumes, such as acetyl cedrene, amylcinnamaldehyde, allylamyl glycolate, β-ionone, Iso E Super, isobutylquinoline, iris oil, irone, indole, ylang ylang oil, undecanal, undecenal, γ-undecalactone, estragole, eugenol, oakmoss, opoponax resinoid, orange oil, eugenol, aurantiol, galaxolide, carvacrol, L-carvone, camphor, canon, carrot seed oil, clove oil, methyl cinnamate, geraniol, geranyl nitrile, isobornyl acetate, geranyl acetate, dimethylbenzylcarbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydroj asmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinen, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexylcinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methylnonylacetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, and various essential oils.

Preferred examples of colors, coloring agents, and dyes include certified colors, such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green. No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407, and Yellow No. 5; other acid dyes, such as Acid Red 14; basic dyes, such as Arianor Sienna Brown, Arianor Madder Red, Arianor Steel Blue, and Arianor Straw Yellow; nitro dyes, such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2, and Basic Blue 26; disperse dyes; natural colors and dyes, for example, anthraquinones, such as astaxanthin and alizarin, naphthoquinones, such as anthocyanidin, β-carotene, catenal, capsanthin, chalcone, carthamin, quercetin, crocin, chlorophyll, curcumin, cochineal, and shikonin, bixin, flavones, betacyanidine, henna, hemoglobin, lycopene, riboflavin, and rutin; oxidation dye intermediates and couplers, such as p-phenylenediamine, toluene-2,5-diamine, o-, m-, or p-aminophenol, m-phenylenediamine, 5-amino-2-methylphenol, resorcin, 1-naphthol, and 2,6-diaminopyridine, and salts thereof; autoxidation dyes, such as indoline; and dihydroxyacetone.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphedramine hydrochloride, and chlorpheniramine maleate; and plant extracts, such as peach leaf extract and Artemisia princeps leaf extract.

Preferred examples of anti-asthmatic agents, anti-chronic obstructive pulmonary disease agents, anti-allergic agents, and immunomodulators include aminophylline, theophyllines, steroids (e.g., fluticasone and beclomethasone), leukotriene antagonists, thromboxane inhibitors, Intal, β2 agonists (e.g., formoterol, salmeterol, albuterol, tulobuterol, clenbuterol, and epinephrine), tiotropium, ipratropium, dextromethorphan, dimemorfan, bromhexine, tranilast, ketotifen, azelastine, cetirizine, chlorpheniramine, mequitazine, tacrolimus, ciclosporin, sirolimus, methotrexate, cytokine modulators, interferon, omalizumab, and protein/antibody formulations.

Preferred examples of anti-infective agents and antifungal agents include oseltamivir, zanamivir, and itraconazole. The anti-pollution material may contain, besides the aforementioned ingredients, known cosmetic ingredients, known pharmaceutical ingredients, and known food ingredients, such as ingredients described in Japanese Standards of Cosmetic Ingredients, Japanese Cosmetic Ingredients Codex, List of Cosmetics Ingredients Japanese Labeling Names issued by the Japan Cosmetic Industry Association, INCI dictionary (The International Cosmetic Ingredient Dictionary and Handbook), Japanese Standards of Quasi-drug Ingredients, Japanese Pharmacopoeia, Japanese Pharmaceutical Excipients, Japan's Specifications and Standards for Food Additives, and other standards, and ingredients described in Japanese and foreign patent publications and patent application publications (including Japanese translations of PCT international application publications and re-publications of PCT international publications) classified as International Patent Classification IPC classes A61K7 and A61K8, in known combinations and in known proportions and amounts.

The anti-pollution material of the present invention may be in any form, so long as it can form a film (layer) on the skin or the surface of hair.

Examples of the form of the anti-pollution material include, but are not limited to, an emulsion form such as oil-in-water (O/W), water-in-oil (W/O), W/O/W, or O/W/O form, an oily form, a solid form, a liquid form, a kneaded form, a stick form, a volatile oil form, a powdery form, a jelly form, a gel form, a paste form, an emulsified polymer form, a sheet form, a mist form, and a spray form. The anti-pollution material may be in any product form, and may be used in the form of, for example, dispersion, emulsion, cream, pack, spray, or gel.

The anti-pollution material may contain any ingredient known to those skilled in the art for achieving the aforementioned form or product form.

### [Production Method for Anti-Pollution Material]

The anti-pollution material of the present invention can be produced through, for example, the following procedure: at least one lipidic peptide compound is mixed with water and, if necessary, an additional ingredient under heating with stirring; and then the resultant mixture is allowed to stand still to cool to about room temperature.

No particular limitation is imposed on the aforementioned heating/stirring temperature, so long as the respective ingredients can be homogeneously mixed. For example, the stirring temperature may be 50°C to 90°C or 60°C to 90°C, for example, 70°C or 80°C, and the stirring time may be appropriately selected from a range of, for example, five minutes to three hours.

The present invention is also directed to a method for preventing pollution of the surface of skin or hair, the method comprising a film formation step of forming a film, on the surface of skin or hair, from an anti-pollution material containing the aforementioned lipidic peptide compound.

The present invention is also directed to a method for preventing adhesion of dust, pollen, particulate matter, gaseous matter, or odorous matter to the surface of skin or hair, the method comprising a film formation step of forming a film, on the surface of skin or hair, from an anti-pollution material containing the aforementioned lipidic peptide compound.

The above-detailed anti-pollution material can be used in the aforementioned method for preventing pollution of the surface of skin or hair and the aforementioned method for preventing adhesion of dust, pollen, particulate matter, gaseous matter, or odorous matter to the surface of skin or hair.

The anti-pollution material of the present invention exhibits its effects by forming appropriate asperities (hereinafter may be referred to as "roughness") on the surface of skin or hair. The roughness is represented by the difference between the maximum height and the minimum height in a direction perpendicular to the surface of skin or hair. The roughness is measured with, for example, an atomic force microscope (AFM).

The anti-pollution material of the present invention forms an average surface roughness of, for example, 3 nm to 500 nm, more preferably, 10 nm to 300 nm.

The anti-pollution material of the present invention is formed by a fiber structure, and the average diameter of the fiber is preferably 10 nm to 100 nm. The average diameter of the fiber can be calculated from a surface image of the anti-pollution material prepared through detection of secondary electrons with a scanning microscope.

### Examples

The present invention will next be described in detail with reference to examples and comparative examples. However, the present invention should not be construed as being limited to the following examples.

### [Synthesis Example 1: Synthesis of Lipidic Peptide (N-Palmitoyl-Gly-His)]

The lipidic peptide compound used in the examples was synthesized by the method described below.

A 500-mL four-necked flask was charged with 14.2 g (91.6 mmol) of histidine, 30.0 g (91.6 mmol) of N-palmitoyl-Gly-methyl, and 300 g of toluene, and 35.3 g (183.2 mmol) of a 28% methanol solution of sodium methoxide serving as a base was added to the flask. The mixture was heated to 60°C in an oil bath and continuously stirred for one hour. Thereafter, the oil bath was removed, and the mixture was allowed to cool to 25°C. To the resultant solution was added 600 g of acetone for reprecipitation, and the precipitate was separated by filtration. The resultant solid was dissolved in a mixed solution of 600 g of water and 750 g of methanol. To the solution was added 30.5 mL (183.2 mmol) of 6N hydrochloric acid to thereby neutralize the solution and precipitate a solid, and the solid was filtered. Subsequently, the resultant solid was dissolved in a mixture of 120 g of tetrahydrofuran and 30 g of water at 60°C, and 150 g of ethyl acetate was added to the solution. The resultant mixture was cooled from 60°C to 30°C, and then the precipitated solid was filtered. The resultant solid was dissolved in a solvent mixture of 120 g of tetrahydrofuran and 60 g of acetonitrile. The solution was heated to 60°C, stirred for one hour, and then cooled, followed by filtration. The resultant solid was washed with 120 g of water, filtered, and then dried under reduced pressure, to thereby produce 26.9 g of a free form of N-palmitoyl-Gly-His (hereinafter may be referred to simply as "Pal-GH") as white crystals (yield: 65%).

### [Preparation Example 1: Preparation of Anti-Pollution Material Sample]

Ingredients were added to a 200-mL beaker (available from HARIO Co., Ltd.) in proportions shown in Table 1 below, and the resultant mixture was heated at a temperature of 75°C with stirring for 10 minutes, to thereby prepare a homogeneous solution. After completion of the heating with stirring, the mixture was cooled with stirring at room temperature until the temperature of the mixture reached 40°C, to thereby prepare a sample of Example 1 (hereinafter may be referred to as "Formulation Example 1"), a sample of Example 2 (hereinafter may be referred to as "Formulation Example 2"), and a sample of Comparative Example (hereinafter may be referred to as "Comparative Formulation 1"). In the aforementioned process, the stirring was performed at 200 rpm.

**Table 1**

| Ingredient | Example 1 | Example 2 | Comparative Example |
|---|---|---|---|
| Pal-GH | 0.01 g | 0.05 g | - |
| 1,2-Hexanediol^{∗1} | 0.004 g | 0.02 g | - |
| Polyoxyethylene lauryl ether^{∗2} | 0.008 g | 0.04 g | - |
| Stearic acid^{∗3} | 0.001 g | 0.005 g | - |
| Purified water | 99.977 g | 99.885 g | 100 g |
| Total | 100 g | 100 g | 100 g |

| | | | |
|---|---|---|---|
| ^{∗}1: available from ITO ^{∗}2: available from Nikko Chemicals Co., Ltd. [trade name: NIKKOL BL-4.2] ^{∗}3: available from Kao Corporation [trade name: LUNAC S-98] | | | |

### [Test Example] Anti-Pollution Test with Bioskin

Bioskin cheek skin model No. 10F (available from Beaulax Co., Ltd.) was cut into a piece having a length of 2 cm and a width of 0.75 cm, and 200 µL of each of the samples of Formulation Example 1, Formulation Example 2, and Comparative Formulation 1 was applied to the cut piece and uniformly spread thereon. Thereafter, the sample-applied piece was allowed to stand still in a thermostatic chamber at 32°C for an hour, and drying of the piece was determined through visual observation.

As a model of PM 2.5 particles, 2.0 g of Carbon ECP-600 JD (available from Lion Corporation, primary particle diameter: 34.0 nm) was weighed in a 50-mL sample tube, and the Bioskin piece on which the sample of Example or Comparative Example was applied and dried was placed into the sample tube three times. Thereafter, excess Carbon ECP-600 JD was shaken off, and then the state of the Bioskin piece was determined.

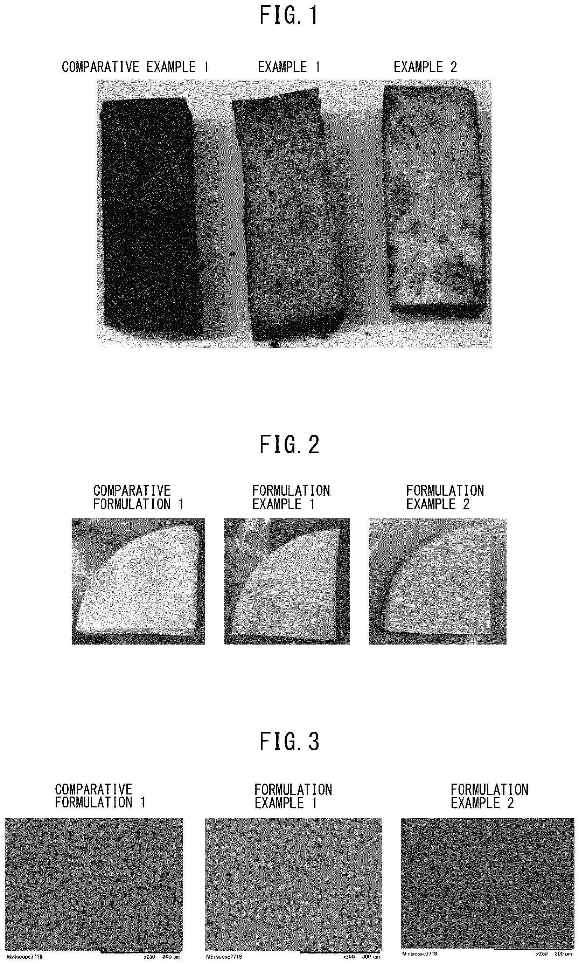

The results are shown in FIG. 1. As compared with the sample of Comparative Formulation 1, the sample of Formulation Example 1 inhibited adhesion of Carbon ECP-600 JD, and the sample of Formulation Example 2 was found to have a higher adhesion inhibiting effect. Thus, the samples of the Examples were found to have an anti-pollution effect.

### [Test for Inhibition of Pollen Adhesion to Artificial Skin Model]

A high-performance artificial skin model Bioskin No. 10F (available from Beaulax Co., Ltd.) was equally cut into four pieces, and 500 µL of each of the solutions of Formulation Example 1, Formulation Example 2, and Comparative Formulation 1 was applied to an equally cut piece, followed by drying in a thermostatic chamber at 32°C for one hour.

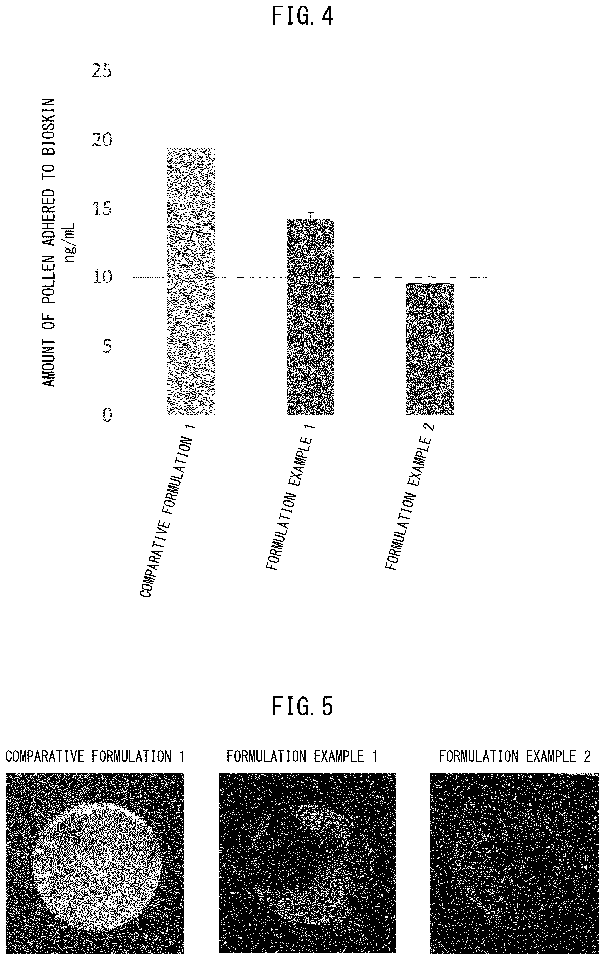

Subsequently, 2.0 g of cedar pollen (available from ITEA) was placed in a scale pan (4 cm × 4 cm), and each of the above-prepared Bioskin pieces was brought into contact with the cedar pollen. Thereafter, the Bioskin piece was pressed with tweezers 10 times, pulled up, and then shaken for 10 seconds, to thereby remove excessively adhered cedar pollen. The cedar pollen adhered to the Bioskin piece was then determined through visual observation (the results are shown in FIG. 2). FIG. 3 shows the results of observation with a scanning electron microscope (SEM) [Miniscope (registered trademark) TM 3000 (available from Hitachi High-Technologies Corporation). FIG. 4 shows the results of quantification with an ITEA cedar pollen allergen (Cryj 1) ELISA kit. As compared with the sample of Comparative Formulation 1, the sample of Formulation Example 1 inhibited pollen adhesion, and the sample of Formulation Example 2 was found to have a higher adhesion inhibiting effect. Thus, the samples of the Examples were found to have an anti-pollution effect.

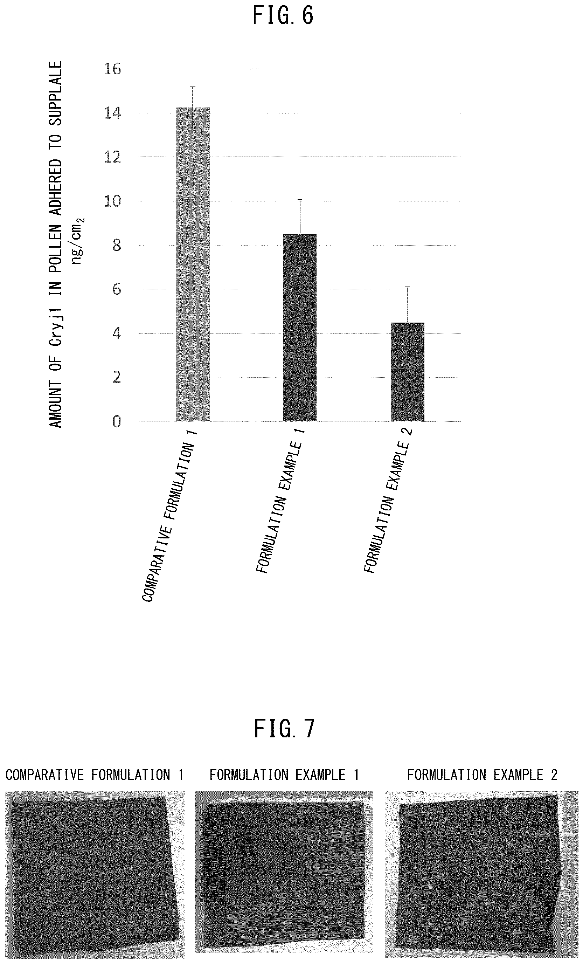

An artificial leather SUPPLALE (available from Idemitsu Technofine Co., Ltd.) was cut into a piece (2 cm × 2 cm), and 1.0 mL of each of the solutions of Formulation Example 1, Formulation Example 2, and Comparative Formulation 1 was applied to the cut piece, followed by drying in a thermostatic chamber at 32°C for one hour. Subsequently, 1.5 g of cedar pollen (available from ITEA) was placed in a 10-mL LABORAN screw tube bottle, and the bottle was covered with the above-prepared SUPPLALE piece. The bottle was inverted so that the pollen came into contact with the SUPPLALE piece, to thereby adhere the pollen to the piece. FIG. 5 shows the results of visual observation of the pollen adhered to the SUPPLALE piece. FIG. 6 shows the results of quantification with an ITEA cedar pollen allergen (Cryj 1) ELISA kit. As compared with the sample of Comparative Formulation 1, the sample of Formulation Example 1 inhibited pollen adhesion, and the sample of Formulation Example 2 was found to have a higher adhesion inhibiting effect. Thus, the samples of the Examples were found to have an anti-pollution effect.

### [Inhibition of Adhesion of PM 2.5 Particles to Artificial Leather]

An artificial leather SUPPLALE (available from Idemitsu Technofine Co., Ltd.) was cut into a piece (2 cm × 2 cm), and 1.0 mL of each of the solutions of Formulation Example 1, Formulation Example 2, and Comparative Formulation 1 was applied to the cut piece, followed by drying in a thermostatic chamber at 32°C for one hour.

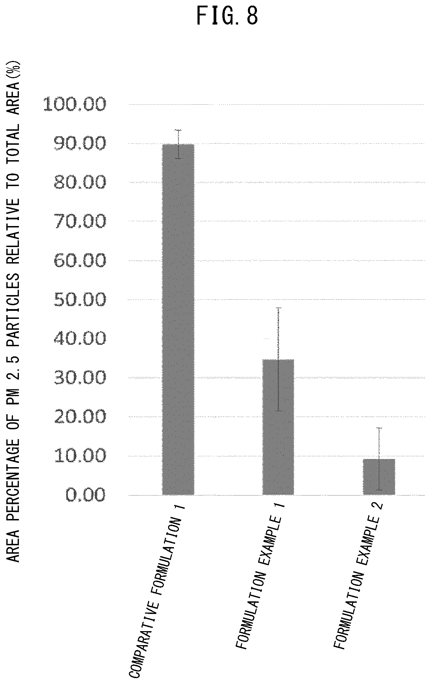

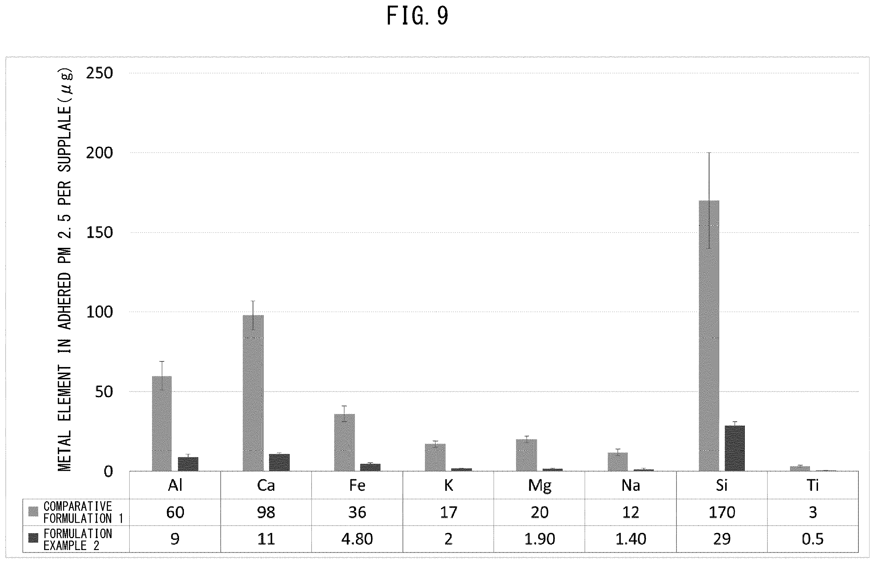

Subsequently, 1.5 g of PM 2.5 particles (NIES-CRM No. city's airborne particulate matter) were placed in a scale pan (4 cm × 4 cm), and each of the above-prepared SUPPLALE pieces was brought into contact with the PM 2.5 particles. Thereafter, the SUPPLALE piece was pressed with tweezers 10 times, pulled up, and then shaken for 10 seconds, to thereby remove excessively adhered PM 2.5 particles. The PM 2.5 particles adhered to the SUPPLALE piece were determined through visual observation (the results are shown in FIG. 7). The adhered PM 2.5 particles were colored through image processing with Microscope VHX-2000 (available from KEYENCE CORPORATION), and the percentage of the area of the colored portion relative to the total area of the image was calculated (the results are shown in FIG. 8). After removal of excessively adhered PM 2.5 particles, metal elements contained in the PM 2.5 particles were quantified with an ICP-emission spectrophotometer, to thereby determine the amount of the adhered PM 2.5 particles (the results are shown in FIG. 9). As compared with the sample of Comparative Formulation 1, the sample of Formulation Example 1 inhibited adhesion of PM 2.5 particles, and the sample of Formulation Example 2 was found to have a higher adhesion inhibiting effect. Thus, the samples of the Examples were found to have an anti-pollution effect.

### [Inhibition of Adhesion of PM 2.5 Particles to Artificial Leather]

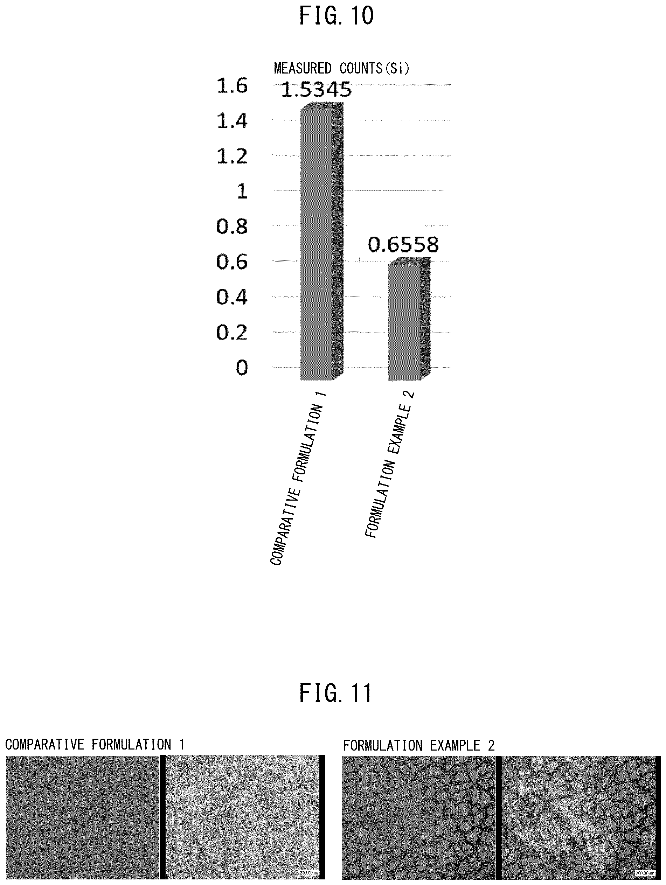

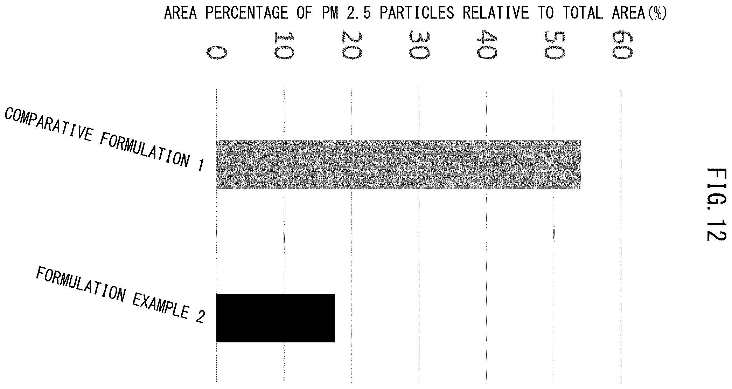

An artificial leather SUPPLALE (available from Idemitsu Technofine Co., Ltd.) was cut into a piece (2 cm × 2 cm), and 1.0 mL of each of the solutions of Formulation Example 2 and Comparative Formulation 1 was applied to the cut piece, followed by drying in a thermostatic chamber at 32°C for one hour. A hole was provided in the side of a 50-mL centrifuge tube, and 100 mg of PM 2.5 particles were placed in the centrifuge tube. The lid of the centrifuge tube was removed, and the centrifuge tube was covered with the above-prepared SUPPLALE piece. Thereafter, air was blown with an air duster through the side hole into the centrifuge tube, to thereby adhere the particles to the SUPPLALE piece by agitation of the particles. The amount of the PM 2.5 particles adhered to the SUPPLALE piece was determined through analysis of silicon (Si) contained in the PM 2.5 particles with an energy dispersive X-ray fluorescence analyzer (EDX-8000, available from SHIMADZU CORPORATION) (the results are shown in FIG. 10). FIG. 11 shows the results of observation of the adhered PM 2.5 particles with Microscope VHX-2000 (available from KEYENCE CORPORATION). The PM 2.5 particles in the observed image were colored through image processing, and the percentage of the area of the colored portion relative to the total area of the image was calculated (the results are shown in FIG. 12). As compared with the sample of Comparative Formulation 1, the sample of Formulation Example 2 inhibited adhesion of PM 2.5 particles. Thus, the sample of the Formulation Example 2 was found to have an anti-pollution effect.

### [Inhibition of Adhesion of PM 2.5 Particles (by Spraying of Compressed Air) to Artificial Leather]

An artificial leather SUPPLALE (available from Idemitsu Technofine Co., Ltd.) was cut into a piece (2 cm × 2 cm), and 1.0 mL of each of the solutions of Formulation Example 2 and Comparative Formulation 1 was applied to the cut piece, followed by drying in a thermostatic chamber at 32°C for one hour.

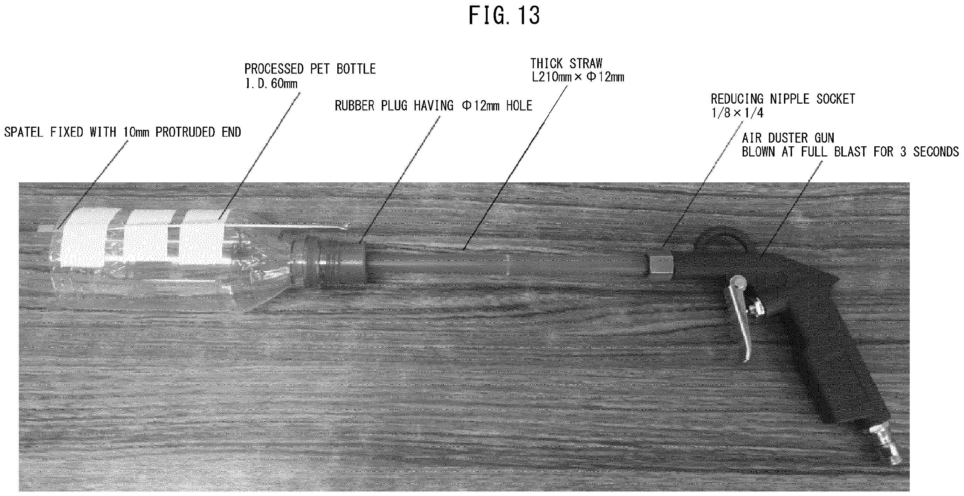

Subsequently, 5.00 mg of PM 2.5 particles (NIES-CRM No. city's airborne particulate matter) were blown onto the SUPPLALE piece with compressed air (FIG. 13).

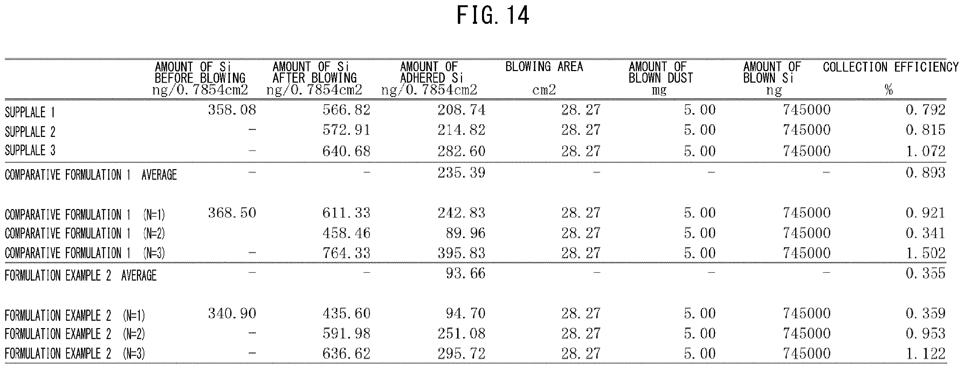

Thereafter, the SUPPLALE piece was recovered, and silicon contained in the PM 2.5 particles was quantified with an energy dispersive X-ray fluorescence analyzer EDX-8000 (available from SHIMADZU CORPORATION). The results are shown in FIG. 14. As compared with the sample of Comparative Formulation 1, the sample of Formulation Example 2 inhibited adhesion of PM 2.5 particles. Thus, the sample of the Formulation Example 2 was found to have an anti-pollution effect.

### [Inhibition of Adhesion of PM 2.5 Particles to Artificial Leather]

An artificial leather SUPPLALE (available from Idemitsu Technofine Co., Ltd.) was cut into a piece (2 cm × 2 cm), and each of the solutions of Formulation Example 2 and Comparative Formulation 1 was sprayed from a spray container to the cut piece, followed by drying in a thermostatic chamber at 32°C for 10 minutes. Each solution was sprayed from the spray container at one push.

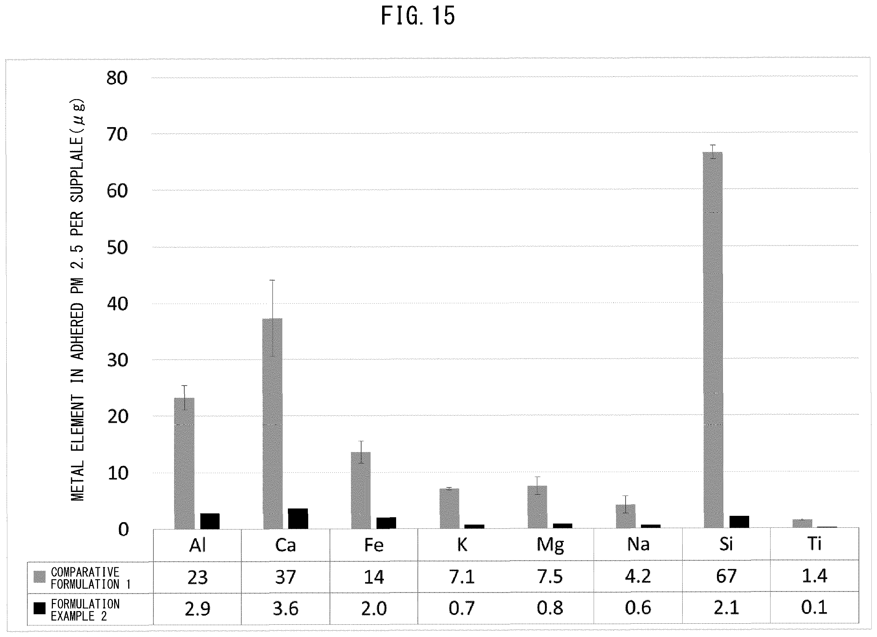

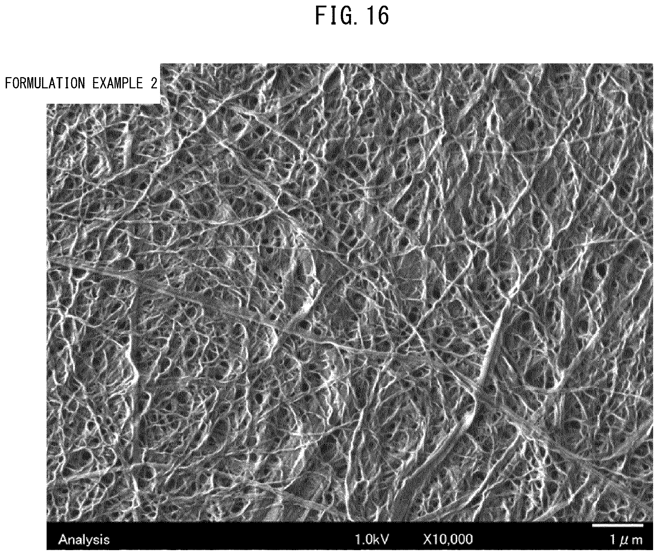

Subsequently, 1.5 g of PM 2.5 particles (NIES-CRM No. city's airborne particulate matter) were placed in a scale pan (4 cm × 4 cm), and each of the above-prepared SUPPLALE pieces was brought into contact with the PM 2.5 particles. Thereafter, the SUPPLALE piece was pressed with tweezers 10 times, pulled up, and then shaken for 10 seconds, to thereby remove excessively adhered PM 2.5 particles. Thereafter, metal elements contained in the PM 2.5 particles were quantified with an ICP-emission spectrophotometer, to thereby determine the amount of the adhered PM 2.5 particles. The results are shown in FIG. 15. A carbon tape was attached to the surface of the SUPPLALE piece to which the solution of Formulation Example 2 was sprayed, to thereby peel a film formed on the SUPPLALE piece, and the film was observed with a field emission scanning electron microscope JSM-7400F (available from JEOL Ltd.) (the results are shown in FIG. 16).

### [Inhibition of Adhesion of Mites to Artificial Leather]

An artificial leather SUPPLALE (available from Idemitsu Technofine Co., Ltd.) was cut into a piece (2 cm × 2 cm), and each of the solutions of Formulation Example 2 and Comparative Formulation 1 was sprayed from a spray container to the cut piece, followed by drying in a thermostatic chamber at 32°C for 10 minutes. Each solution was sprayed from the spray container at one push.

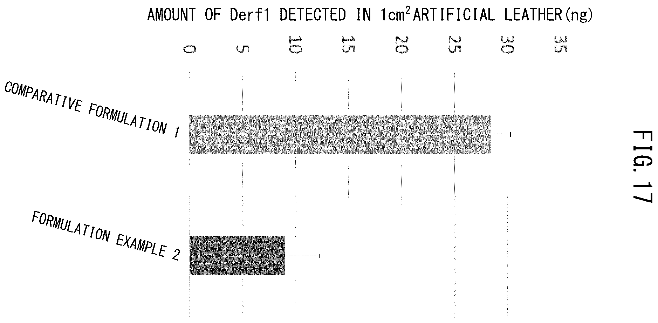

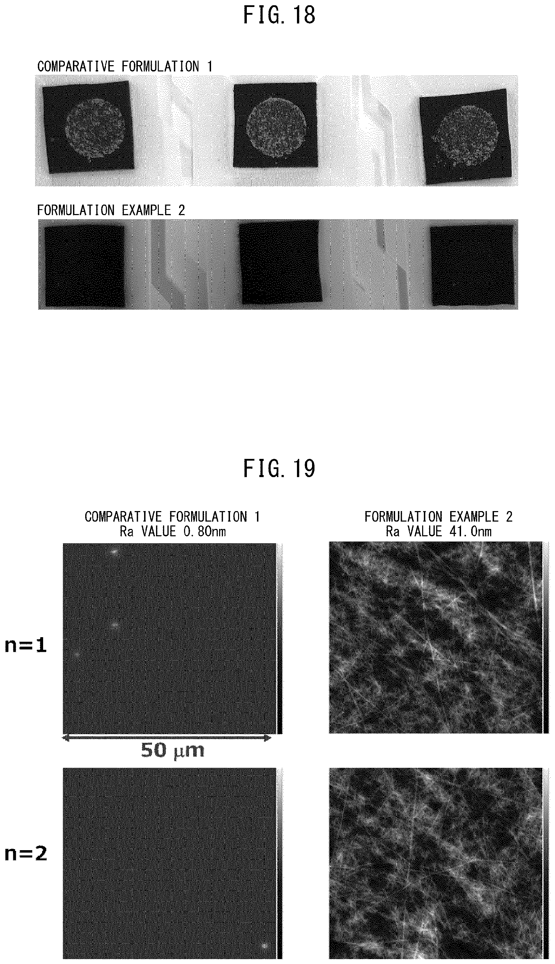

Subsequently, 0.5 g of mites (*Dermatophagoides farinae*) (available from Biostir Inc.) were placed in a 10-mL LABORAN screw tube bottle, and the bottle was covered with the above-prepared SUPPLALE piece. The bottle was inverted so that the mites came into contact with the SUPPLALE piece, to thereby adhere the mites to the piece. Thereafter, the amount of the adhered mites (*Dermatophagoides farinae*) was determined by quantification of Derf1 adhered to the SUPPLALE piece with an ITEA mite (Derf1) high sensitivity ELISA kit. The results are shown in FIG. 17. As compared with the sample of Comparative Formulation 1, the sample of Formulation Example 2 was found to inhibit adhesion of the mites (*Dermatophagoides farinae*). FIG. 18 is a photograph of *Dermatophagoides farinae* adhered to the SUPPLALE pieces.

### [Measurement of Roughness of Coating Film]

Each of the solutions of Formulation Example 2 and Comparative Formulation 1 was applied to a silicon wafer, and then dried in a thermostatic chamber at 32°C for 10 minutes. The roughness of the film formed on the surface of the silicon wafer was measured with an atomic force microscope (AFM) Dimension Icon (available from Bruker AXS) (the results are shown in FIG. 19). The average roughness of the film was 0.80 nm in Comparative Formulation 1. In contrast, the average roughness of the film was 41.0 nm in Formulation Example 2; i.e., the average roughness in Formulation Example 2 was larger than that in Comparative Formulation 1. The results suggested that the film formed from the sample of Formulation Example 2 can inhibit adhesion of a pollutant.

### [Formulation Examples 3 to 8 and Comparative Formulation 2: Inhibition of Adhesion of Pollen to Artificial Leather]

### [Preparation of Sample]

According to the table shown below, ingredients heated to 75°C were stirred in a 200-mL beaker (available from HARIO Co., Ltd.), and the resultant mixture was heated at 75°C with stirring for 10 minutes. After the elapse of 10 minutes, the mixture was cooled with stirring at room temperature until the temperature of the mixture reached 40°C. In the aforementioned process, the stirring was performed at 200 rpm.

**Table 2**

| Ingredient (wt%) | | | Comparative Formulation 2 | Formulation Example 3 | Formulation Example 4 | Formulation Example 5 | Formulation Example 6 | Formulation Example 7 | Formulation Example 8 |
|---|---|---|---|---|---|---|---|---|---|
| Composition [1] | Pal-GH | 3 wt% | - | 0.05 g | 0.05 g | 0.05 g | 0.05 g | 0.05 g | 0.05 g |
| | Cetanol^{∗1} | 0.2 wt% | - | 0.003 g | 0.003 g | 0.003 g | 0.003 g | 0.003 g | 0.003 g |
| | Myristyl alcohol^{∗2} | 0.2 wt% | - | 0.003 g | 0.003 g | 0.003 g | 0.003 g | 0.003 g | 0.003 g |
| | Lauryl hydroxy sulfobetain^{∗3} | 6 wt% | - | 0.1 g | 0.1 g | 0.1 g | 0.1 g | 0.1 g | 0.1 g |
| | 1,3-Butylene glycol | 20 wt% | | 0.33 g | 0.33 g | 0.33 g | 0.33 g | 0.33 g | 0.33 g |
| | Purified water | 70.6 wt% | - | 1.176 g | 1.176 g | 1.176 g | 1.176 g | 1.176 g | 1.176 g |
| | Composition [1] total | 100 wt% | 0 | 1.662 g | 1.662 g | 1.662 g | 1.662 g | 1.662 g | 1.662 g |
| | Purified water | | 99.9 g | 98.188 g | 98.138 g | 98.088 g | 98.038 g | 97.988 g | 97.938 g |
| | Citric acid | | 0.1 g | 0.1 g | 0.1 g | 0.1 g | 0.1 g | 0.1 g | 0.1 g |
| | Arginine | | | 0.05 g | 0.1 g | 0.15 g | 0.2 g | 0.25 g | 0.30 g |
| Total | | | 100 g | 100 g | 100 g | 100 g | 100 g | 100 g | 100 g |

| | | | | | | | | | |
|---|---|---|---|---|---|---|---|---|---|
| ^{∗}1: available from Kao Corporation [trade name: KALCOL 6098] ^{∗}2: available from Kao Corporation [trade name: KALCOL 4098] ^{∗}3: available from Kao Corporation [trade name: AMPHITOL 20HD] ^{∗}4: available from Daicel Corporation | | | | | | | | | |

### [Inhibition of Adhesion of Pollen to Artificial Leather]

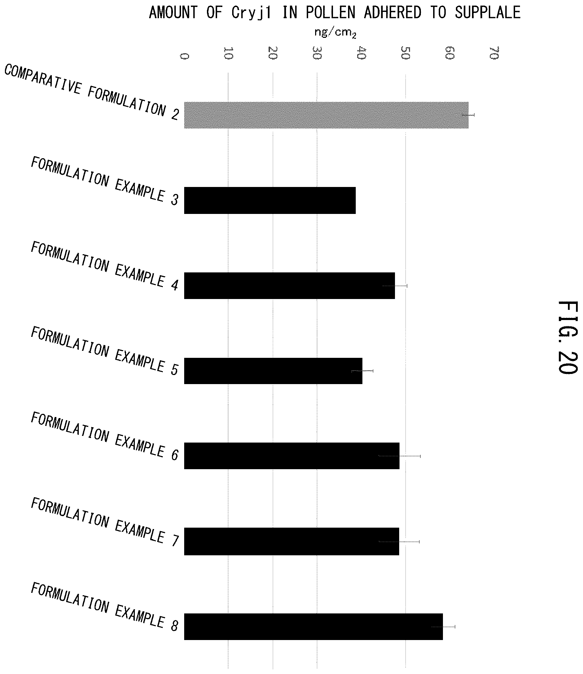

An artificial leather SUPPLALE (available from Idemitsu Technofine Co., Ltd.) was cut into a piece (2 cm × 2 cm), and 1.0 mL of each of the solutions of Comparative Formulation 2 and Formulation Examples 3 to 8 was applied to the cut piece, followed by drying in a thermostatic chamber at 32°C for one hour. Subsequently, 1.5 g of cedar pollen (available from ITEA) was placed in a 10-mL LABORAN screw tube bottle, and the bottle was covered with the above-prepared SUPPLALE piece. The bottle was inverted so that the pollen came into contact with the SUPPLALE piece, to thereby adhere the pollen to the piece. FIG. 20 shows the results of quantification with an ITEA cedar pollen allergen (Cryj 1) ELISA kit. As compared with the sample of Comparative Formulation 2, the samples of Formulation Examples 3 to 8 inhibited pollen adhesion. Thus, the samples of the Examples were found to have an anti-pollution effect.

## Claims

1. An anti-pollution material **characterized by** comprising a lipidic peptide compound wherein a lipidic moiety having a C₁₀₋₂₄ aliphatic group is bonded to a peptide moiety formed of at least two identical or different amino acid repeats.

2. The anti-pollution material according to claim 1, wherein the anti-pollution material can form an anti-pollution film on the surface of skin or hair.

3. The anti-pollution material according to claim 2, wherein the anti-pollution material prevents adhesion of dust, pollen, particulate matter, mites (including carcasses thereof), gaseous matter, or odorous matter to the surface of skin or hair.

4. The anti-pollution material according to any one of claims 1 to 3, wherein the lipidic peptide compound contains at least one of compounds of the following Formulae (1) to (3) or pharmaceutically usable salts of the compounds: 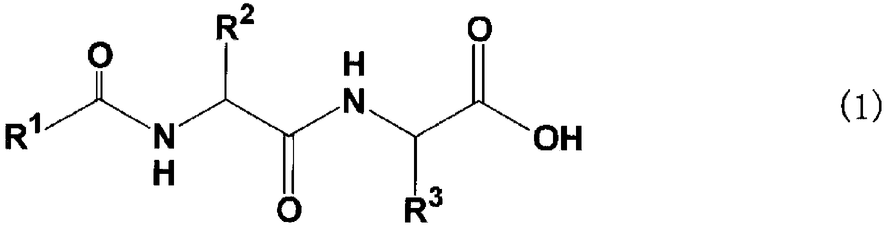 (wherein R¹ is a C₉₋₂₃ aliphatic group; R² is a hydrogen atom, or a C₁₋₄ alkyl group having, if necessary, a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring group having, if necessary, one to three nitrogen atoms, or a fused heterocyclic ring group composed of the 5-membered ring and the 6-membered ring), 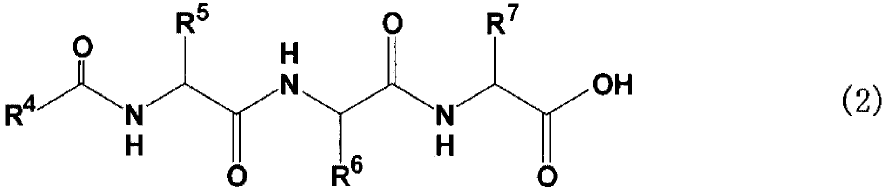 (wherein R⁴ is a C₉₋₂₃ aliphatic group; and R⁵ to R⁷ are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring), and 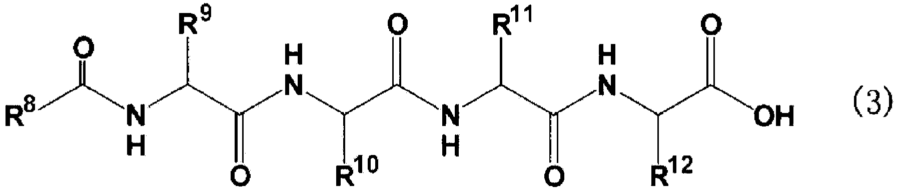 (wherein R⁸ is a C₉₋₂₃ aliphatic group; and R⁹ to R¹² are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring).

5. A method for preventing pollution of the surface of skin or hair, the method comprising a film formation step of forming a film, on the surface of skin or hair, from an anti-pollution material containing a lipidic peptide compound wherein a lipidic moiety having a C₁₀₋₂₄ aliphatic group is bonded to a peptide moiety formed of at least two identical or different amino acid repeats.

6. The method according to claim 5, wherein the lipidic peptide compound contains at least one of compounds of the following Formulae (1) to (3) or pharmaceutically usable salts of the compounds: 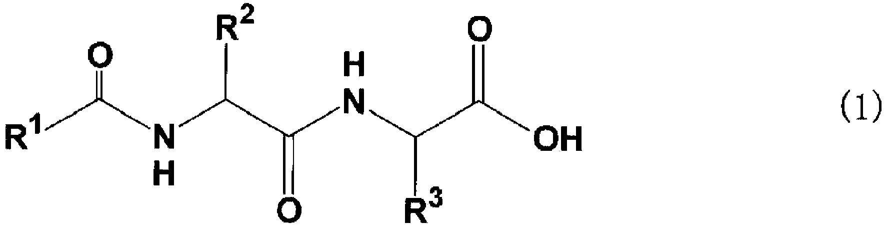 (wherein R¹ is a C₉₋₂₃ aliphatic group; R² is a hydrogen atom, or a C₁₋₄ alkyl group having, if necessary, a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring group having, if necessary, one to three nitrogen atoms, or a fused heterocyclic ring group composed of the 5-membered ring and the 6-membered ring), 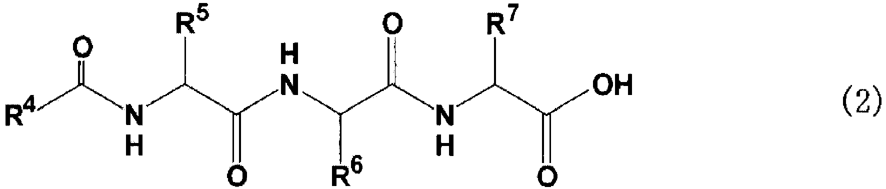 (wherein R⁴ is a C₉₋₂₃ aliphatic group; and R⁵ to R⁷ are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring), and 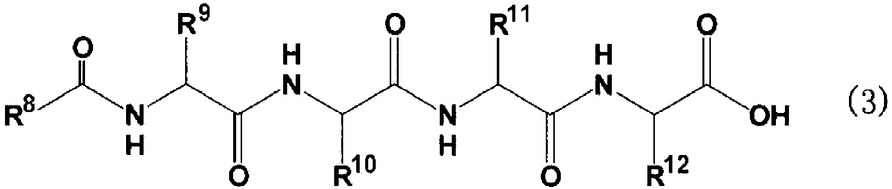 (wherein R⁸ is a C₉₋₂₃ aliphatic group; and R⁹ to R¹² are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring).

7. A method for preventing adhesion of dust, pollen, mites (including carcasses thereof), particulate matter, gaseous matter, or odorous matter to the surface of skin or hair, the method comprising a film formation step of forming a film, on the surface of skin or hair, from an anti-pollution material containing a lipidic peptide compound wherein a lipidic moiety having a C₁₀₋₂₄ aliphatic group is bonded to a peptide moiety formed of at least two identical or different amino acid repeats.

8. The method according to claim 7, wherein the lipidic peptide compound contains at least one of compounds of the following Formulae (1) to (3) or pharmaceutically usable salts of the compounds: 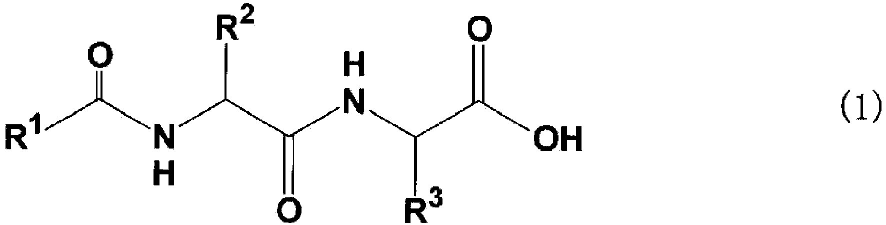 (wherein R¹ is a C₉₋₂₃ aliphatic group; R² is a hydrogen atom, or a C₁₋₄ alkyl group having, if necessary, a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring group having, if necessary, one to three nitrogen atoms, or a fused heterocyclic ring group composed of the 5-membered ring and the 6-membered ring), 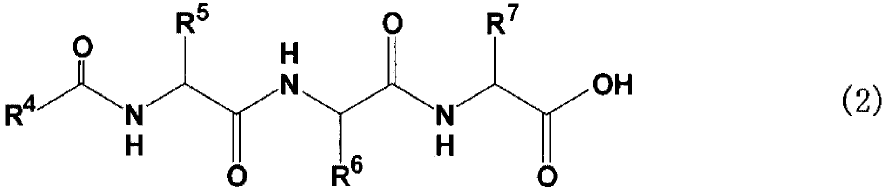 (wherein R⁴ is a C₉₋₂₃ aliphatic group; and R⁵ to R⁷ are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring), and 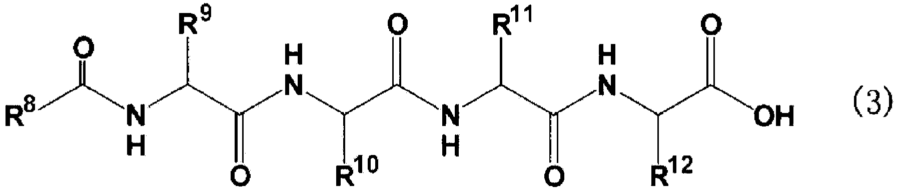 (wherein R⁸ is a C₉₋₂₃ aliphatic group; and R⁹ to R¹² are each independently a hydrogen atom, a C₁₋₄ alkyl group having, if necessary, a C₁ or C₂ branched chain, or a -(CH₂)ₙ-X group, wherein n is a number of 1 to 4, and X is an amino group, a guanidino group, a - CONH₂ group, or a 5-membered ring or 6-membered ring group having, if necessary, one to three nitrogen atoms, or a fused heterocyclic ring group composed of the 5-membered ring and the 6-membered ring).

9. The anti-pollution material according to any one of claims 1 to 4, wherein a film formed from the material has a surface roughness, and the average surface roughness is 3 nm to 500 nm.
